(19) Europäisches Patentamt
European Patent Office
Office européen des brevets

(11) EP 2 781 217 A1

(12) # EUROPEAN PATENT APPLICATION

(43) Date of publication:
**24.09.2014 Bulletin 2014/39**

(51) Int Cl.:
***A61K 31/575*** (2006.01) ***A61P 3/10*** (2006.01)

(21) Application number: **13159736.1**

(22) Date of filing: **18.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **ETH Zurich**
**8092 Zürich (CH)**

(72) Inventors:
- **Wolfrum, Christian**
**8104 Weiningen ZH (CH)**
- **Carreira, Erick**
**8126 Zumikon (CH)**
- **Cribiu, Riccardo**
**8057 Zürich (CH)**

(74) Representative: **Rentsch Partner AG**
**Rechtsanwälte und Patentanwälte**
**Fraumünsterstrasse 9**
**Postfach 2441**
**8022 Zürich (CH)**

(54) **ROR gamma modulators**

(57) The present invention relates to compounds of formula I, or a pharmaceutically acceptable salt thereof,

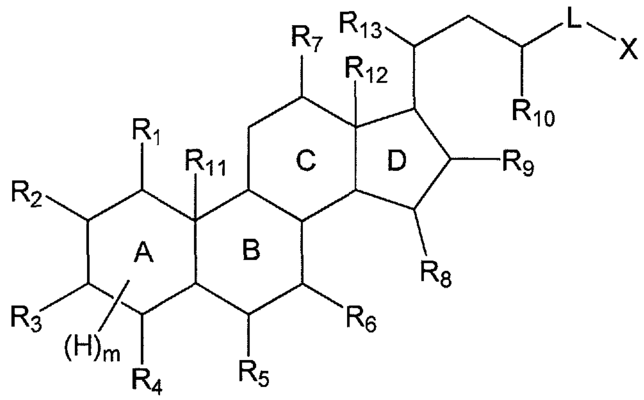

I

characterized in that at least one of $R_8$, $R_9$, $R_{10}$ is $-OR_a$; for use in the treatment or prevention, suppression or amelioration of a disease mediated by the ROR gamma receptor in a subject in need thereof, in particular diabetes and diabetes-related disorders, specifically type II diabetes, methods of their production, as well as methods of treatment or prevention of such diseases.



Printed by Jouve, 75001 PARIS (FR)

## Description

### Field of the Invention

The present invention relates to compounds for use in the treatment or prevention, suppression or amelioration of a disease mediated by the ROR gamma receptor in a subject in need thereof, in particular diabetes and diabetes-related disorders, specifically type II diabetes, methods of production of such compounds, as well as methods of treatment or prevention of such diseases.

### Background

Worldwide, there are about 250 million people that suffer from diabetes (type I and type II) and the number is projected to double in next two decades. Type 1 diabetes is based on lack of insulin production by the pancreas. Although the causes are not entirely known, type 1 diabetes is a multi-factorial autoimmune disease that results from the specific and progressive destruction of insulin producing beta-cells in the pancreas. Typical treatment of type 1 diabetes includes (multiple) administration of insulin, which however does not cure diabetes or prevent its eventual effects such as kidney failure, blindness, nerve damage, amputations, heart attack and stroke. Even with insulin treatment, type 1 diabetes usually results in a drastic reduction in quality of life and shortens the average life span by 15 years. Type 2 diabetes, also called non-insulin dependent diabetes mellitus, is is a heterogeneous disease characterized by abnormalities in carbohydrate and fat metabolism. The causes of type 2 diabetes are multi-factorial and include both genetic and environmental elements that affect beta-cell function and insulin sensitivity in tissues such as muscle, liver, pancreas and adipose tissue. As a consequence impaired insulin secretion is observed and paralleled by a progressive decline in beta-cell function and chronic insulin resistance. The inability of the endocrine pancreas to compensate for peripheral insulin resistance leads to hyperglycaemia and onset of clinical type 2 diabetes characterized by hyperglycemia, insulin resistance, absolute or relative insulin deficiency, hyperglucagonemia, and increased hepatic glucose production. However, there is still no definitive treatment for the disease.

Applicants have now surprisingly found that the retinoid-receptor related orphan receptors (ROR) may act as a central regulator of adipogenesis. The retinoid-receptor related orphan receptors consist of three family members, namely ROR alpha (Becker-Andree, Biochem. Biophys. Res. Commun. 1993, 194:1371), ROR beta (Andre et al., Gene 1998, 516:277) and ROR gamma (He et al, Immunity 1998, 9:797) and constitute the NR1F (ROR /RZR) subgroup of the nuclear receptor superfamily (Mangelsdorf et al., Cell 1995, 83:835). Applicants have shown that in particular the ROR gamma receptor, which has been linked exclusively to immunological functions, may inhibit adipogenesis and may allow protection from diet or genetically induced insulin resistance.

Thus the present invention provides compounds of the invention, in particular compounds based on a polyhydroxylated cholane skeleton, that are able to act as ROR (gamma) receptor modulators or ligands, thereby influencing the biological pathways in adipogenesis controlled by ROR (gamma), and thus may be useful for the prevention, treatment and amelioration of diabetes and diabetes-related disorders, in particular type II diabetes.

### Summary of the Invention

The present invention relates in a first aspect to compounds based on a polyhydroxylated cholane skeleton and pharmaceutically acceptable salts or stereoisomers thereof for use as modulators of the ROR receptor, in particular, as selective ROR gamma receptor ligands (also hereinafter called compounds of the invention or ROR gamma receptor ligands or modulators of the present invention) having the general formula I

I

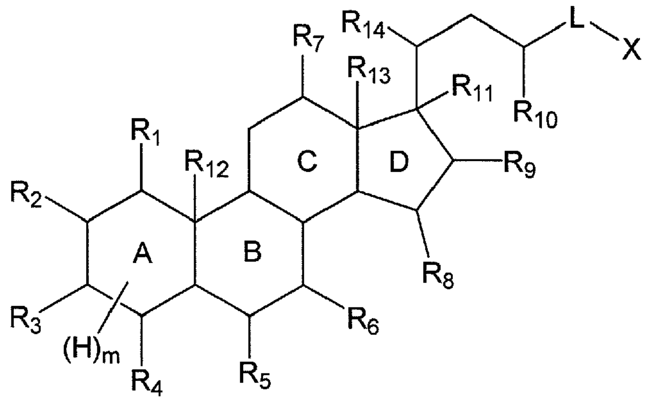

I

wherein

| | |
|---|---|
| $R_1$, $R_2$, | are independently of each other H, hal, -$OR_a$, -$SR_a$, -$NR_aR_b$, -$COOR_a$, -CON-$R_aR_b$, wherein $R_a$ and $R_b$ are independently of each other H or (C1-6)alkyl, or $R_1$, $R_2$ form together with the C-atoms to which they are linked an epoxy group; |
| $R_3$, $R_4$, $R_5$, $R_6$, $R_7$, $R_8$, $R_9$, $R_{10}$, $R_{11}$ | are independently of each other H, hal, -$OR_a$, -$SR_a$, -$NR_aR_b$, - $COOR_a$, -CON-$R_aR_b$, oxo, thio wherein $R_a$ and $R_b$ are independently of each other H or (C1-6)alkyl, with the proviso that at least one of $R_8$, $R_9$, $R_{10}$, $R_{11}$ is -$OR_a$; |
| $R_{12}$, $R_{13}$, $R_{14}$ | are independently of each other H, (C1-10)alkyl, wherein one or more non neighbouring $CH_2$ groups may be replaced with -O-, -S-, -CO-, -CO-O-, -O-CO-, -$NR_a$-, -CO-$NR_a$-, -$NR_a$-CO-, -C=C-, or -C≡C-; wherein $R_a$ is H or (C1-6)alkyl; |
| L | is a linking group, such as a covalent bond or a straight-chain or branched C(1-12)alkyl, which is unsubstituted or substituted by at least one CN, hal, OH, $NR_aR_b$, $COOR_a$, $NO_2$, and wherein one or more of the non-adjacent $CH_2$ groups may independently be replaced by a group selected from -O-, -CO-, -CO-O-, -O-CO-, -$NR_a$-, -$NR_a$-CO-, -CO-$NR_a$-, - CH=CH-, -C≡C-, wherein $R_a$ and $R_b$ are independently of each other H or C(1-6)alkyl, |
| X | is H, -$OR_a$, -$SR_a$, -$NR_aR_b$, -$COOR_c$, -$CONR_aR_c$; wherein $R_a$ and $R_b$ are independently of each other H or (C1-6)alkyl and $R_c$ is -H, -(C1-6)alkyl, -NH-$(CH_2)_n$-$CO_2R_a$ or -NH-$(CH_2)$p-$SO_3R_a$, wherein n, p is 1, 2, or 3 and $R_a$ is -H or -(C1-6)alkyl; |
| m | is 0 to 5. |

In specific embodiments, the compounds of the invention are based on a polyhydroxylated cholane skeleton having a -$C_X$-L-X- substituent at C17, and wherein the hydroxyl (or oxo-) substituents are preferably in one or more positions selected from C1, C3, C6, C7, C12, C15, C16, C17, C23, more preferably wherein the hydroxyl (or oxo-) substituents are at a total of three or four positions selected from C1, C3, C6, C7, C12, C15, C16, C17, C23, most preferably wherein the hydroxyl (or oxo-) substituents are at a total of three or four positions selected from C1, C3, C6, C7, C12, C15, C16, C17, C23, with the proviso that at least one hydroxyl (or oxo) substituent is in position C15 or C16 or C17 or C23.

In some embodiments, the invention includes compounds of formula II and pharmaceutically acceptable salts or stereoisomers thereof, preferably its stereoisomeric form IIa,

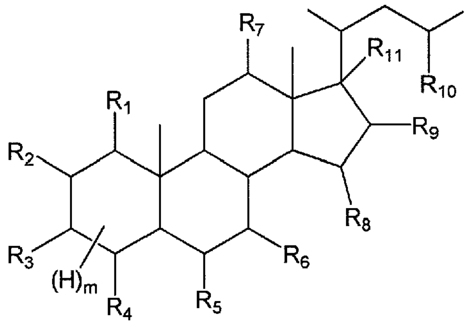 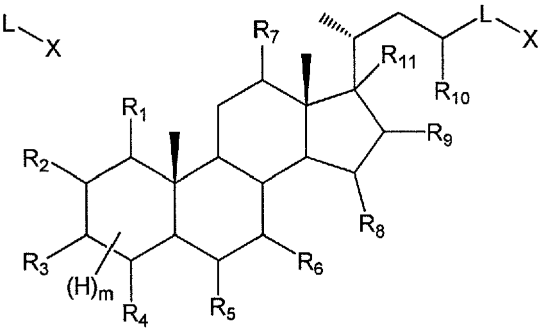

II IIa

wherein

| | |
|---|---|
| $R_1$, $R_2$, | are independently of each other H, hal, $-OR_a$, $-SR_a$, $-NR_aR_b$, $-COOR_a$, $-CONR_aR_b$, wherein $R_a$ and $R_b$ are independently of each other H or (C1-6)alkyl, or $R_1$, $R_2$ form together with the C-atoms to which they are linked an epoxy group; |
| $R_3$, $R_5$, $R_6$, $R_7$, $R_8$, $R_9$, $R_{10}$, $R_{11}$ | are independently of each other H, hal, $-OR_a$, $-SR_a$, $-NR_aR_b$, $- COOR_a$, $-CONR_aR_b$, oxo, thio wherein $R_a$ and $R_b$ are independently of each other H or (C1-6)alkyl, with the proviso that at least one of $R_8$, $R_9$, $R_{10}$, $R_{11}$, is $-OR_a$; |
| $R_4$ | is H, hal, |
| L | is a linking group, such as a covalent bond or a straight-chain or branched C(1-12)alkyl, which is unsubstituted or substituted by at least one CN, hal, OH, $NR_aR_b$, $COOR_a$, $NO_2$, and wherein one or more of the non-adjacent $CH_2$ groups may independently be replaced by a group selected from -O-, -CO-, -CO-O-, -O-CO-, $-NR_a-$, $-NR_a-CO-$, $-CO-NR_a-$, - CH=CH-, -C≡C-, wherein $R_a$ and $R_b$ are independently of each other H or C(1-6)alkyl, |
| X | is H, $-OR_a$, $-SR_a$, $-NR_aR_b$, $-COOR_c$, $-CONR_aR_c$; wherein $R_a$ and $R_b$ are independently of each other H or (C1-6)alkyl and $R_c$ is -H, -(C1-6)alkyl, $-NH-(CH_2)_n-CO_2R_a$ or $-NH-(CH_2)p-SO_3R_a$, wherein n, p is 1, 2, or 3 and $R_a$ is -H or -(C1-6)alkyl; |
| m | is 0 to 5. |

Preferably at least three groups, preferably three or four groups, selected from $R_3$, $R_4$, $R_5$, $R_6$, $R_7$, $R_8$, $R_9$, $R_{10}$ and $R_{11}$ are $-OR_a$ or oxo, with the proviso that at least one of $R_8$, $R_9$, $R_{10}$, $R_{11}$ is $-OR_a$.

Thus, specific compounds of the invention are tri-hydroxylated compounds having

- two hydroxyl (or oxo) substituents at two positions selected from C1, C3, C6, C7, and C12, and one hydroxyl (or oxo) substituent at either C15, C16, C17 or C23; or
- one hydroxyl (or oxo) substituent at either C1, C3, C6, C7, or C12, and two hydroxyl (or oxo) substituents ate either (i) C15 and C16 or (ii) C15 and C17 or (iii) C15 and C23 or (iv) C16 and C17 or (v) C16 and C23 or (vi) C17 and C23; or
- three hydroxyl (or oxo) substituents at either (i) C15, C16, C17 or (ii) C15, C16, C23 or (iii) C15, C17, C23 or (iv) C16, C17, C23.

  Other specific compounds of the invention are tetra-hydroxylated compounds having
- three hydroxyl (or oxo) substituents at three positions selected from C1, C3, C6, C7, and C12, and one hydroxyl (or oxo) substituent at either C15, C16, C17 or C23; or
- two hydroxyl (or oxo) substituents at two positions selected from C1, C3, C6, C7, and C12, and two hydroxyl or (oxo-substituents) at either (i) C15 and C16 or (ii) C15 and C17 or (iii) C15 and C23 or (iv) C16 and C17 or (v) C16 and C23 or (vi) C17 and C23; or
- one hydroxyl (or oxo) substituent at either C1, C3, C6, C7, or C12, and three hydroxyl (or oxo) substituents at either (i) C15, C16, C17 or (ii) C15, C16, C23 or (iii) C15, C17, C23 or (iv) C16, C17, C23; or

four hydroxyl (or oxo) substituents at C15, C16, C17 and C23.

In preferred embodiments, L is a covalent bond or a straight-chain or branched C(1-12)alkyl, which is unsubstituted and wherein one or more of the non-adjacent $CH_2$ groups may independently be replaced by a group selected from -O-, -CO-, - CO-O-, preferably straight-chain or branched C(1-12)alkyl.

In a further aspect, the methods of invention also relates to convenient and efficient methods of synthesis of the compounds of the invention.

The present invention also relates in yet a further aspect to pharmaceutical compositions comprising at least one compound of the present invention and a pharmaceutically acceptable carrier, and optionally at least one further therapeutically active agent.

In yet another aspect, the present invention relates to methods for the treatment or prevention of disorders, diseases, or conditions responsive to the modulation of the ROR gamma receptor in a mammal in need thereof, in particular diabetes and diabetes-related disorders, specifically type II diabetes, by administering at least one compound (or a pharmaceutical composition thereof) of the present invention.

In yet a further aspect, the present invention relates to the kits comprising at least one compound of the present invention (or a pharmaceutical composition thereof).

**Brief Description of Figures**

Figure 1. Adipocyte differentiation assay in the presence of a tetraol compound of the invention. The Y-axis is differentiation (in %). The X axis is the amount of 3a,7$\beta$,15$\alpha$,(23R)-tetrahydroxy-5$\beta$-24-oxocholanyl-amino ethansulfonic acid (indicated as "tetraol") in $\mu$M.

**Detailed Description of the Invention**

Unless specified otherwise, the following terms have the indicated meanings:

The term "subject" means a mammal, such as a human or an animal, being either male or female, preferably a human.

The term "ligand" or "modulator" refers to a natural or synthetic compound which binds a receptor molecule to form a receptor-ligand complex. The term ligand may include agonists, antagonists, and compounds with partial agonist/antagonist action. An "agonist" is a natural or synthetic compound which binds the receptor to form a receptor-agonist complex thereby activating said receptor, initiating a pathway signaling and further biological processes. By "antagonist" is meant a natural or synthetic compound that has a biological effect opposite to that of an agonist. An antagonist binds the receptor and blocks the action of a receptor agonist. The term "ligand" or "modulator", when used according to the present invention in combination with e.g. a ROR (gamma) receptor, refers to a(n) (endogenous) compound that can interact with a ROR (gamma) receptor and initiate a pharmacological or biochemical response.

The term "binding affinity" refers to the ability of a compound to bind to its biological target. For the present invention, it refers to the ability of a compound of the invention, to bind to the ROR (gamma) receptor.

The term "efficacy" describes the relative intensity of response induced by a compound when binding to its receptor. Maximal response depends on the efficiency of coupling to the receptor.

The term "diabetes" as used herein includes both insulin-dependent diabetes mellitus (i.e., IDDM, also known as type I diabetes) and non-insulin-dependent diabetes mellitus (i.e., NIDDM, also known as type II diabetes). Type I diabetes, or insulin-dependent diabetes, is the result of an absolute deficiency of insulin, the hormone which regulates glucose utilization. Type II diabetes, or insulin-independent diabetes (i.e., non-insulin-dependent diabetes mellitus), often occurs in the face of normal, or even elevated levels of insulin and appears to be the result of the inability of tissues to respond appropriately to insulin. The compounds and compositions of the present invention may be useful for treating both type I and type II diabetes, but may be especially effective for treating type II diabetes (as shown hereinafter).

The term "diabetes related disorders" as used herein includes diseases, disorders and conditions that are related to type 1 and type 2 diabetes, in particular diseases, disorders and conditions that are related to type 2 diabetes, and therefore may be treated, controlled or in some cases prevented, by administration of the compounds and compositions of this invention. Diabetes related disorders include e.g. hyperglycemia, low glucose tolerance, insulin resistance, obesity, lipid disorders, dyslipidemia, hyperlipidemia, hypertriglyceridemia, hypercholesterolemia, low HDL levels, high LDL levels, atherosclerosis, vascular restenosis, irritable bowel syndrome, inflammatory bowel disease, including Crohn's disease and ulcerative colitis, other inflammatory conditions, pancreatitis, abdominal obesity, neurodegenerative disease, retinopathy, nephropathy, neuropathy, Syndrome X, ovarian hyperandrogenism (polycystic ovarian syndrome), and other disorders where insulin resistance is a contributive component.

"Treatment" of diabetes according to the invention refers to the administration of at least one compound of the present invention to treat diabetes and diabetes-related disorders as defined herein, e.g. in a subject in need thereof, e.g. a diabetic subject. Possible outcomes of such treatment may be decreasing the glucose level in a subject with elevated glucose levels and/or improving glycemic control and/or decreasing insulin levels in a subject with elevated insulin levels and/or to reduce an increased plasma glucose concentration and/or to reduce an increased insulin concentration and/or to reduce an increased blood triglyceride concentration and/or to increase insulin sensitivity and/or enhancing glucose tolerance in a subject with glucose intolerance and/or to reduce insulin resistance and/or to lower

plasma insulin levels and/or an improvement in glycemic control, particularly in type 2 diabetes.

"Prevention" (or "prophylaxis") of diabetes according to the invention refers to the administration of at least one compound of the present invention to prevent or treat the onset of diabetes and diabetes-related disorders as defined herein in a subject in need thereof, e.g. a prediabetic subject.

The term "subject" as used herein refers to an animal, preferably a mammal, most preferably a human.

The term "polyhydroxylated cholane skeleton" includes in particular polyhydroxylated cholestanes and more specifically polyhydroxylated bile acids. A polyhydroxylated cholane or bile acid compound according to the invention includes, but is not limited to, from trihydroxylated, tetrahydroxylated, pentahydroxylated, hexahydroxylated bile acids, etc., up to the maximal hydroxylation level, but preferably (at least) trihydroxylated and (at least) tetrahydroxylated bile acids. The term "bile acid" encompasses all naturally occurring (chemically synthesized) bile acids (whether from man or from another animal) including conjugates thereof (e.g. in particular conjugates with glycine, taurine and possibly other amino acids), as well as synthetic or semi-synthetic analogs. Most naturally occurring bile acids are characterized by hydroxyl groups in the A, B, and C ring of the cholane skeleton, predominantly at one or more of positions C3, C7, C12, possibly (but more unusual) at positions C1, C6 and others. The junction of rings A and B in the cholane skeleton (and in bile acids) exists in two isomeric forms, i.e. the C5- and C8-substituents are cis (5-beta-cholane) or trans (5-alphacholane) configured. For the compounds of the present invention, if not specified differently, both the alpha- and beta-isomer of the A/B-ring junction are contemplated, preferably the beta-isomer.

The present invention relates in a first aspect to compounds for use as modulators of the ROR receptor, in particular, as selective ROR gamma receptor ligands, having the general formula I or pharmaceutically acceptable salts or stereoisomers thereof

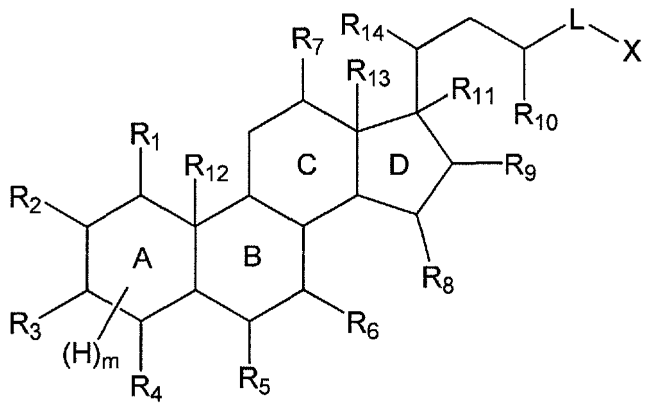

I

wherein

| | |
|---|---|
| $R_1$, $R_2$, | are independently of each other H, hal, $-OR_a$, $-SR_a$, $-NR_aR_b$, $-COOR_a$, $-CONR_aR_b$, wherein $R_a$ and $R_b$ are independently of each other H or (C1-6)alkyl, or $R_1$, $R_2$ form together with the C-atoms to which they are linked an epoxy group; $R_3$, $R_4$, $R_5$, $R_6$, $R_7$, $R_8$, $R_9$, $R_{10}$, $P_{11}$ are independently of each other H, hal, $-OR_a$, $-SR_a$, $-NR_aR_b$, - $COOR_a$, $-CONR_aR_b$, oxo, thio wherein $R_a$ and $R_b$ are independently of each other H or (C1-6)alkyl, with the proviso that at least one of $R_8$, $R_9$, $R_{10}$, $R_{11}$ is $-OR_a$; |
| $R_{12}$, $R_{13}$, $R_{14}$ | are independently of each other H, (C1-10)alkyl, wherein one or more non neighbouring $CH_2$ groups may be replaced with -O-, -S-, -CO-, -CO-O-, -O-CO-, $-NR_a-$, $-CO-NR_a-$, $-NR_a-CO-$, -C=C-, or -C≡C-; wherein $R_a$ is H or (C1-6)alkyl; |
| L | is a linking group, such as a covalent bond or straight-chain or branched C(1-12)alkyl, which is unsubstituted or substituted by at least one CN, hal, OH, $NR_aR_b$, $COOR_a$, $NO_2$ and wherein one or more of the non-adjacent $CH_2$ groups may independently be replaced by a group selected from -O-, -CO-, -CO-O-, -O-CO-, $-NR_a-$, $-NR_a-CO-$, $-CO-NR_a-$, - CH=CH-, -C≡C-, wherein $R_a$ and $R_b$ are independently of each other H or C(1-6)alkyl, |
| X | is H, $-OR_a$, $-SR_a$, $-NR_aR_b$, $-COOR_c$, $-CONR_aR_c$; wherein $R_a$ and $R_b$ are independently of each other H or (C1-6)alkyl and $R_c$ is -H, -(C1-6)alkyl, $-NH-(CH_2)_n-CO_2R_a$ or $-NH-(CH_2)p-SO_3R_a$, wherein n, p is 1, 2, or 3 and $R_a$ is -H or -(C1-6)alkyl; |
| m | is 0 to 5. |

It is understood, that $(H)_m$ represents all hydrogen substituents on the indicated A-ring (i.e. on C1, C2, C3, C4, C5 and C10). The skilled person will know how many H-substituents are present for a specific substitution pattern of groups $R_1$, $R_2$, $R_{2'}$, $R_3$ on ring A. For example, m = 5 may represent a fully saturated ring having no double bonds, m = 3 may represent e.g. a ring having one double bond (between e.g. C1-C2 or between C4-C5), m = 2 may represent e.g. a ring having one double bond (between e.g. C1-C2 or between C4-C5) and $R_3$ being oxo, and m = 0 may represent e.g. a fully unsaturated ring having double bonds between C1-C2 and between C4-C5 and $R_3$ being oxo. Thus the degree of unsaturation and nature of substituents determines the number of H-atoms present or vice versa, the number of H-atoms is a way to indicate the degree of unsaturation of ring A.

In specific embodiments, the compounds of the invention are based on a polyhydroxylated cholane skeleton according to formula I, and wherein the hydroxyl (or oxo-) substituents are preferably in one or more positions selected from C1, C3, C6, C7, C12, C15, C16, C17, C23, more preferably wherein the hydroxyl (or oxo-) substituents are at least three positions selected from C1, C3, C6, C7, C12, C15, C16, C17, C23, most preferably wherein the hydroxyl (or oxo-) substituents are at a total of three or four positions selected from C1, C3, C6, C7, C12, C15, C16, C17, C23, with the proviso that at least one hydroxyl (or oxo) substituent is in position C15 or C16 or C17 or C23.

Thus, in specific embodiments, the compounds of the invention have three or four hydroxyl (or oxo-substituents) one of which is at position C15 or C16 or C17 or C23, characterized by e.g. the following hydroxylation patterns:

(i) a hydroxyl substituent at C15 in combination with further hydroxyl (or oxo-) substituents at position C7, preferably C1 and C7, C3 and C7, C6 and C7, or C12 and C7, and

(ii) a hydroxyl substituent at C16 in combination with further hydroxyl (or oxo-) substituents at position C7, preferably C1 and C7, C3 and C7, C6 and C7, or C12 and C7, and

(iii) a hydroxyl substituent at C17 in combination with further hydroxyl (or oxo-) substituents at position C7, preferably C1 and C7, C3 and C7, C6 and C7, or C12 and C7, and

(iv) a hydroxyl substituent at C23 in combination with further hydroxyl (or oxo-) substituents at position C7, preferably C1 and C7, C3 and C7, C6 and C7, or C12 and C7.

It is understood, that in case of tetrahydroxylated compounds the above hydroxylation patterns (i), (ii), (iii), and (iv) include a fourth hydroxyl group which is at one of the remaining positions selected from C1, C3, C6, C7, C12, C15, C16, C17, and C23.

In other embodiments, the compounds of the invention have three or four hydroxyl (or oxo-substituents), of which two are at positions selected from C15, C16, C17 and C23. Thus, the hydroxylation patterns include for example

(i) hydroxyl substituents at C15 and C16 in combination with further hydroxyl (or oxo-) substituents at position C7, preferably C1 and C7, C3 and C7, C6 and C7, or C12 and C7; and

(ii) hydroxyl substituents at C15 and C17 in combination with further hydroxyl (or oxo-) substituents at position C7, preferably C1 and C7, C3 and C7, C6 and C7, or C12 and C7; and

(iii) hydroxyl substituents at C15 and C23 in combination with further hydroxyl (or oxo-) substituents at position C7, preferably C1 and C7, C3 and C7, C6 and C7, or C12 and C7; and

(iv) hydroxyl substituents at C16 and C17 in combination with further hydroxyl (or oxo-) substituents at position C7, preferably C1 and C7, C3 and C7, C6 and C7, or C12 and C7; and

(v) hydroxyl substituents at C16 and C23 in combination with further hydroxyl (or oxo-) substituents at position C7, preferably C1 and C7, C3 and C7, C6 and C7, or C12 and C7; and

(vi) hydroxyl substituents at C17 and C23 in combination with further hydroxyl (or oxo-) substituents at position C7, preferably C1 and C7, C3 and C7, C6 and C7, or C12 and C7.

In further embodiments, the compounds of the invention are (i) tri-hydroxylated compounds having hydroxyl substituents at either (i) C15, C16, C17 or (ii) C15, C16, C23 or (iii) C15, C17, C23 or (iv) C16, C17, C23, or (ii) tetra-hydroxylated compounds having hydroxyl substituents at positions C15, C16, C17 and C23.

In a preferred embodiment, $R_{12}$, $R_{13}$, $R_{14}$ are independently of each other H, (C1-10)alkyl, more preferably methyl, ethyl, propyl, butyl, most preferably methyl.

In some embodiments, $R_1$, $R_2$ are independently of each other H, hal, $-OR_a$, $-COOR_a$, wherein $R_a$ is H or (C1-6)alkyl, or form together with the C-atoms to which they are linked an epoxy group.
Preferably, $R_2$ is H or hal, or forms together with $R_1$ and the C-atoms to which $R_1$ and $R_2$ are linked an epoxy group.
Preferably $R_4$ is H or hal, more preferably H or C1, Br, I, most preferably H.
Preferably, $R_3$, $R_5$, $R_6$, $R_7$ are independently of each other H, $-OR_a$, $-NR_aR_b$, $-COOR_a$, $-CONR_aR_b$ or oxo, wherein $R_a$ and $R_b$ are independently of each other H or (C1-6)alkyl.
In more preferred embodiments $R_3$, is H, $-OR_a$, $-NR_aR_b$ or oxo, wherein $R_a$ and $R_b$ are independently of each other H or (C1-6)alkyl; $R_5$, $R_6$, $R_7$ are independently of each other H, - $OR_a$ or $-NR_aR_b$, wherein $R_a$ and $R_b$ are independently of each other H or (C1-6)alkyl; and $R_{12}$, $R_{13}$, $R_{14}$ are independently of each other H, (C1-10)alkyl, more preferably methyl, ethyl, propyl, butyl, most preferably methyl.
Thus, preferably, the invention includes compounds of formula II and pharmaceutically acceptable salts or stereoisomers thereof, preferably its stereoisomeric form IIa,

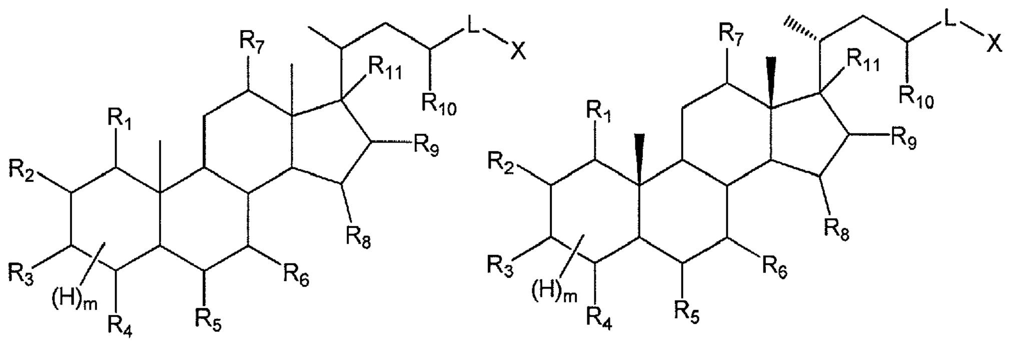

II IIa

wherein

| | |
|---|---|
| $R_1$, $R_2$, | are independently of each other H, hal, $-OR_a$, $-SR_a$, $-NR_aR_b$, $-COOR_a$, $-CONR_aR_b$, wherein $R_a$ and $R_b$ are independently of each other H or (C1-6)alkyl, or $R_1$, $R_2$ form together with the C-atoms to which they are linked an epoxy group; |
| $R_3$, $R_5$, $R_6$, $R_7$, $R_8$, $R_9$, $R_{10}$, $R_{11}$ | are independently of each other H, hal, $-OR_a$, $-SR_a$, $-NR_aR_b$, - $COOR_a$, $-CONR_aR_b$, oxo, thio wherein $R_a$ and $R_b$ are independently of each other H or (C1-6)alkyl, with the proviso that at least one of $R_8$, $R_9$, $R_{10}$, $R_{11}$ is $-OR_a$; |
| $R_4$ | is H, hal, |
| L | is a linking group, such as a covalent bond or a straight-chain or branched C(1-12)alkyl, which is unsubstituted or substituted by at least one CN, hal, OH, $NR_aR_b$, $COOR_a$, $NO_2$ and wherein one or more of the non-adjacent $CH_2$ groups may independently be replaced by a group selected from -O-, -CO-, -CO-O-, -O-CO-, $-NR_a-$, $-NR_a-CO-$, $-CO-NR_a-$, - CH=CH-, -C≡C-, wherein $R_a$ and $R_b$ are independently of each other H or C(1-6)alkyl, |
| X | is H, $-OR_a$, $-SR_a$, $-NR_aR_b$, $-COOR_c$, $-CONR_aR_c$; wherein $R_a$ and $R_b$ are independently of each other H or (C1-6)alkyl and $R_c$ is -H, -(C1-6)alkyl, $-NH-(CH_2)_n-CO_2R_a$ or $-NH-(CH_2)p-SO_3R_a$, wherein n, p is 1, 2, or 3 and $R_a$ is -H or -(C1-6)alkyl; |
| m | is 0 to 5. |

In specific embodiments L (in all of the formulas disclosed herein) is a covalent bond or a straight-chain or branched C(1-12)alkyl, which is unsubstituted and wherein one or more of the non-adjacent $CH_2$ groups may independently be replaced by a group selected from -O-, -CO-, - CO-O-. Preferably L is straight-chain or branched C(1-12)alkyl, more preferably a straight-chain or branched C(1-6)alkyl as defined herein.
Preferably, X (in all of the formulas disclosed herein) is H, $-OR_a$, $-COOR_c$, $-CONR_aR_c$, wherein $R_a$ is H or (C1-6)alkyl and $R_c$ is -H, -(C1-6)alkyl, $-NH-(CH_2)_n-CO_2R_a$ or $-NH-(CH_2)p-SO_3R_a$, wherein n, p is 1, 2, or 3 and $R_a$ is -H or -(C1-6)alkyl. Preferably, $R_c$ is -H, -(C1-6)alkyl, $-NH-CH_2-CO_2R_a$ or $-NH-(CH_2)_2-SO_3R_a$, wherein $R_a$ is -H or -(C1-6)alkyl.
In preferred embodiments, group -L-X (in all of the formulas disclosed herein) is $-(C1\text{-}6)alkyl-COOR_a$, more preferably $-(CH_2)_2-COOR_a$, wherein $R_a$ is -H or -(C1-6)alkyl.
Preferably, $R_2$ is H or hal, or forms together with $R_1$ and the C-atoms to which $R_1$ and $R_2$ are linked an epoxy group.

In a preferred embodiment, at least three groups, preferably three or four groups, selected from $R_3$, $R_5$, $R_6$, $R_7$, $R_8$, $R_9$, $R_{10}$, $R_{11}$ are -$OR_a$ or oxo, with the proviso that at least one of $R_8$, $R_9$, $R_{10}$, $R_{11}$ is -$OR_a$, wherein $R_a$ is H or C(1-6)alkyl.

Thus, in a first specific embodiment compounds are included, wherein (i) $R_8$ and two or three groups selected from $R_3$, $R_5$, $R_6$, $R_7$, $R_9$, $R_{10}$, $R_{11}$ preferably selected from $R_3$, $R_5$, $R_6$, $R_7$ are - $OR_a$ or (ii) $R_9$ and two or three groups selected from $R_3$, $R_5$, $R_6$, $R_7$, $R_8$, $R_{10}$, $R_{11}$ preferably selected from $R_3$, $R_5$, $R_6$, $R_7$ are -$OR_a$ or (iii) $R_{10}$ and two or three groups selected from $R_3$, $R_5$, $R_6$, $R_7$, $R_8$, $R_9$, preferably selected from $R_3$, $R_5$, $R_6$, $R_7$ are -$OR_a$, or (iv) $R_{11}$ and two or three groups selected from $R_3$, $R_5$, $R_6$, $R_7$, $R_8$, $R_9$, $R_{10}$ preferably selected from $R_3$, $R_5$, $R_6$, $R_7$ are -$OR_a$, wherein $R_a$ is H or C(1-6)alkyl.

Thus compounds of the invention include compounds of formula IIIa, IIIb, IIIc, IIId and all pharmaceutically acceptable salts or stereoisomers thereof,

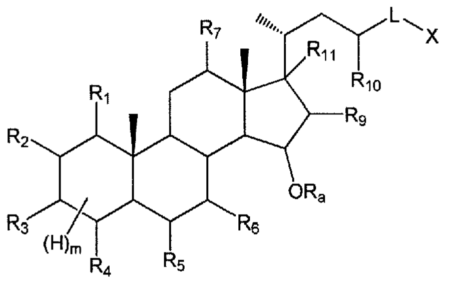

IIIa

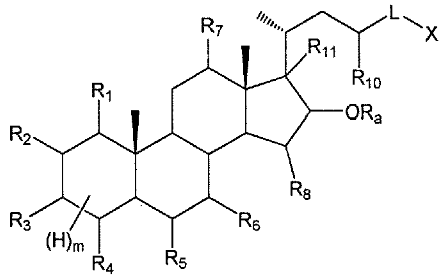

IIIb

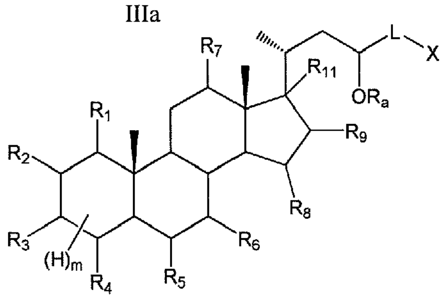

IIIc

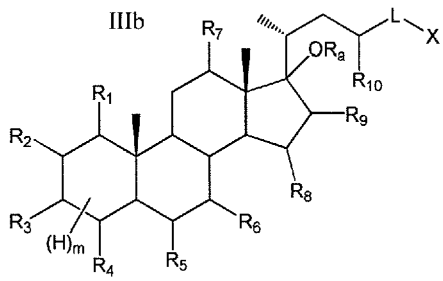

IIId

wherein

| | |
|---|---|
| $R_1$, $R_2$, | are independently of each other H, hal, -$OR_a$, -$SR_a$, -$NR_aR_b$, -$COOR_a$, -$CONR_aR_b$, wherein $R_a$ and $R_b$ are independently of each other H or (C1-6)alkyl, or $R_1$, $R_2$ form together with the C-atoms to which they are linked to an epoxy group; |
| $R_3$, $R_5$, $R_6$, $R_7$, $R_8$, $R_9$, $R_{10}$, $R_{11}$, | are independently of each other H, hal, -$OR_a$, -$SR_a$, -$NR_aR_b$, - $COOR_a$, -$CONR_aR_b$, oxo, thio wherein $R_a$ and $R_b$ are independently of each other H or (C1-6)alkyl, with the proviso that at least one group, preferably two or three groups selected from of $R_1$, $R_2$, $R_3$, $R_4$, $R_5$, $R_6$, $R_7$, $R_8$, $R_9$, $R_{10}$, and $R_{11}$, are -$OR_a$; |
| $R_4$ | is H, hal, |
| L | is a covalent bond or a straight-chain or branched C(1-12)alkyl, which is unsubstituted and wherein one or more of the non-adjacent $CH_2$ groups may independently be replaced by a group selected from -O-, -CO-, - CO-O; |
| X | is H, -$OR_a$, -$SR_a$, -$NR_aR_b$, -$COOR_c$, -$CONR_aR_c$; wherein $R_a$ and $R_b$ are independently of each other H or (C1-6)alkyl and $R_c$ is -H, -(C1-6)alkyl, -NH-$(CH_2)_n$-$CO_2R_a$ or -NH-$(CH_2)$p-$SO_3R_a$, wherein n, p is 1, 2, or 3 and $R_a$ is -H or -(C1-6)alkyl; |
| $R_a$ | is H or C(1-6)alkyl, |
| m | is 0 to 5. |

Preferably, $R_2$ and $R_4$ are H.

Preferred embodiments include (i) compounds of formula IIIa wherein two or three groups selected from of $R_1$, $R_3$, $R_5$, $R_6$, $R_7$, $R_9$, $R_{10}$ and $R_{11}$ are -$OR_a$; (ii) compounds of formula IIIb wherein two or three groups selected from of $R_1$, $R_3$, $R_5$, $R_6$, $R_7$, $R_8$, $R_{10}$ and $R_{11}$ are -$OR_a$; (iii) compounds of formula IIIc wherein two or three groups selected from of $R_1$, $R_3$, $R_5$, $R_6$, $R_7$, $R_8$, $R_9$, and $R_{11}$ are -$OR_a$, (iv) compounds of formula IIId wherein two or three groups selected

from of $R_1$, $R_3$, $R_5$, $R_6$, $R_7$, $R_8$, $R_9$, and $R_{10}$ are $-OR_a$, wherein $R_a$ is H or (C1-6)alkyl.

More preferred are compounds of formula IIIa, IIIb, IIIc, IIId, wherein (i) $R_3$ and $R_6$ are $-OR_a$ or (ii) $R_5$ and $R_6$ are $-OR_a$, wherein $R_a$ is H or (C1-6)alkyl, or (iii) $R_3$ and $R_7$ are $-OR_a$, or (iv) $R_1$ and $R_3$ are $-OR_a$, or (v) $R_1$ and $R_5$ are $-OR_a$, or (vi) $R_1$ and $R_6$ are $-OR_a$, or (vii) $R_1$ and $R_7$ are - $OR_a$, or (viii) $R_6$ and $R_7$ are $-OR_a$, or (ix) $R_3$ and $R_5$ are $-OR_a$, or (x) $R_5$ and $R_7$ are $-OR_a$, wherein $R_a$ is H or (C1-6)alkyl.

Most preferred are compounds of formula IIIa, IIIb, IIIc, IIId, wherein (i) $R_1$, $R_3$ and $R_6$ are - $OR_a$, or (ii) $R_3$, $R_6$ and $R_7$ are $-OR_a$, or (iii) $R_1$, $R_5$ and $R_6$ are $-OR_a$, or (iv) $R_5$, $R_6$ and $R_7$ are - $OR_a$, or (v) $R_1$, $R_3$ and $R_5$ are $-OR_a$, or (vi) $R_3$, $R_5$ and $R_6$ are $-OR_a$, or (vii) $R_1$, $R_3$ and $R_7$ are -

$OR_a$, or (viii) $R_1$, $R_5$ and $R_7$ are $-OR_a$, or (ix) $R_1$, $R_6$ and $R_7$ are $-OR_a$, wherein $R_a$ is H or (C1-6)alkyl.

In a second specific embodiment compounds are included, wherein

(i) $R_8$, $R_9$, and one or two groups selected from $R_3$, $R_5$, $R_6$, $R_7$, $R_{10}$, $R_{11}$ preferably selected from $R_3$, $R_5$, $R_b$, $R_7$ are $-OR_a$ or

(ii) $R_8$, $R_{10}$, and one or two groups selected from $R_3$, $R_5$, $R_6$, $R_7$, $R_9$, $R_{11}$, preferably selected from $R_3$, $R_5$, $R_6$, $R_7$ are $-OR_a$ or

(iii) $R_8$, $R_{11}$, and one or two groups selected from $R_3$, $R_5$, $R_6$, $R_7$, $R_9$, $R_{10}$, preferably selected from $R_3$, $R_5$, $R_6$, $R_7$ are $-OR_a$ or

(iv) $R_9$, $R_{10}$, and one or two groups selected from $R_3$, $R_5$, $R_6$, $R_7$, $R_8$, $R_{11}$, preferably selected from $R_3$, $R_5$, $R_6$, $R_7$ are $-OR_a$, or

(v) $R_9$, $R_{11}$, and one or two groups selected from $R_3$, $R_5$, $R_6$, $R_7$, $R_8$, $R_{10}$, preferably selected from $R_3$, $R_5$, $R_6$, $R_7$ are $-OR_a$ or

(vi) $R_{10}$, $R_{11}$, and one or two groups selected from $R_3$, $R_5$, $R_6$, $R_7$, $R_8$, $R_9$, preferably selected from $R_3$, $R_5$, $R_6$, $R_7$ are $-OR_a$,

wherein $R_a$ is H or C(1-6)alkyl.

Thus compounds of the invention also include compounds of formula Va, Vb or Vc, and all pharmaceutically acceptable salts or stereoisomers thereof,

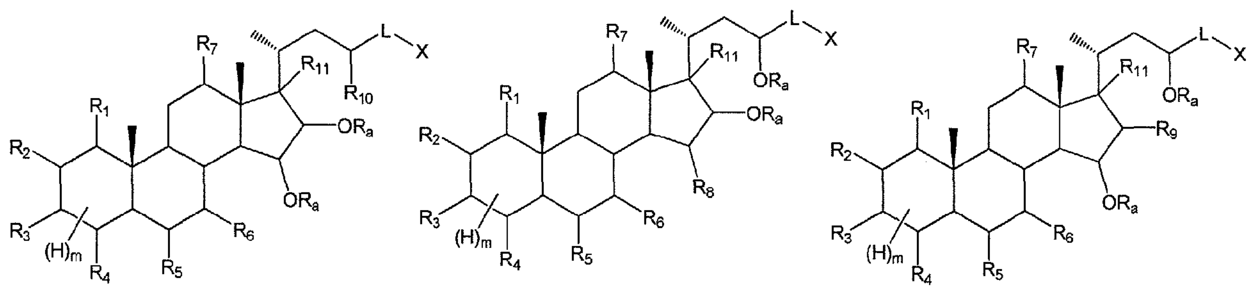

Va Vb Vc

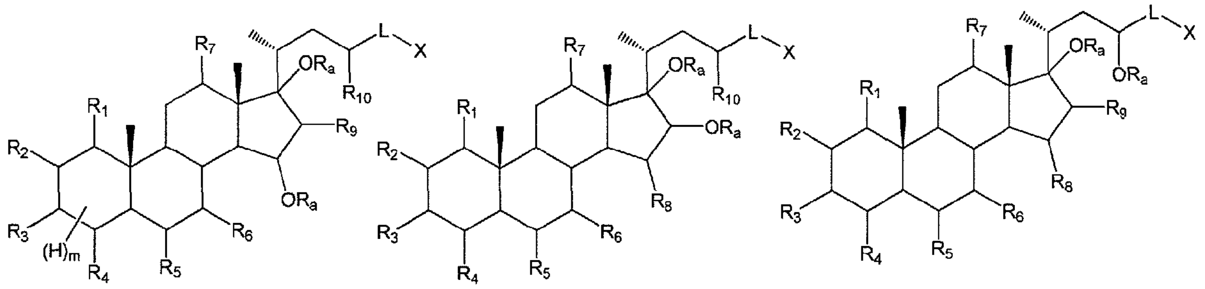

Vd Ve Vf

wherein

| | |
|---|---|
| $R_1$, $R_2$, | are independently of each other H, hal, -$OR_a$, -$SR_a$, -$NR_aR_b$, -$COOR_a$, -$CONR_aR_b$, wherein $R_a$ and $R_b$ are independently of each other H or (C1-6)alkyl, or $R_1$, $R_2$ form together with the C-atoms to which they are linked to an epoxy group; |
| $R_3$, $R_5$, $R_6$, $R_7$, $R_8$, $R_9$, $R_{10}$, $R_{11}$, | are independently of each other H, hal, -$OR_a$, -$SR_a$, -$NR_aR_b$, - $COOR_a$, -$CONR_aR_b$, oxo, thio wherein $R_a$ and $R_b$ are independently of each other H or (C1-6)alkyl, wherein preferably one or two groups selected from of $R_1$, $R_2$, $R_3$, $R_4$, $R_5$, $R_6$, $R_7$, $R_8$, $R_9$, $R_{10}$, and $R_{11}$ are -$OR_a$; |
| $R_4$ | is H, hal, |
| L | is a covalent bond or a straight-chain or branched C(1-12)alkyl, which is unsubstituted and wherein one or more of the non-adjacent $CH_2$ groups may independently be replaced by a group selected from -O-, -CO-, - CO-O; |
| X | is H, -$OR_a$, -$SR_a$, -$NR_aR_b$, -$COOR_c$, -$CONR_aR_c$; wherein $R_a$ and $R_b$ are independently of each other H or (C1-6)alkyl and $R_c$ is -H, -(C1-6)alkyl, -NH-$(CH_2)_n$-$CO_2R_a$ or -NH-$(CH_2)$p-$SO_3R_a$, wherein n, p is 1, 2, or 3 and $R_a$ is -H or -(C1-6)alkyl; |
| $R_a$ | is H or C(1-6)alkyl, |
| m | is 0 to 5. |

Preferably, $R_2$ and $R_4$ are H; $R_1$, $R_3$, $R_5$, $R_b$, $R_7$, $R_8$, $R_9$, $R_{10}$, $R_{11}$, are independently of each other H, hal, -$OR_a$, -$SR_a$, -$NR_aR_b$, -$COOR_a$, -$CONR_aR_b$, oxo, thio wherein $R_a$ and $R_b$ are independently of each other H or (C1-6)alkyl; L is a covalent bond or a straight-chain or branched C(1-12)alkyl; X is H, -$OR_a$, -$COOR_c$, -$CONR_aR_c$, wherein $R_a$ is H or (C1-6)alkyl and $R_c$ is -H, -(C1-6)alkyl, -NH-$(CH_2)_n$-$CO_2R_a$ or -NH-$(CH_2)$p-$SO_3R_a$, wherein n, p is 1, 2, or 3 and $R_a$ is -H or - (C1-6)alkyl, and m is 0 to 5.

Preferred embodiments include (i) compounds of formula Va wherein one or two groups selected from of $R_1$, $R_3$, $R_5$, $R_6$, $R_7$, $R_{10}$, and $R_{11}$ are -$OR_a$; (ii) compounds of formula Vb wherein one or two groups selected from of $R_1$, $R_3$, $R_5$, $R_6$, $R_7$, $R_8$ and $R_{11}$ are -ORa; (iii) compounds of formula Vc wherein one or two groups selected from of $R_1$, $R_3$, $R_5$, $R_6$, $R_7$, $R_9$ and $R_{11}$ are -$OR_a$, (iv) compounds of formula Vd wherein one or two groups selected from of $R_1$, $R_3$, $R_5$, $R_b$, $R_7$, $R_9$ and $R_{10}$ are -$OR_a$, (v) compounds of formula Ve wherein one or two groups selected from of $R_1$, $R_3$, $R_5$, $R_6$, $R_7$, $R_8$ and $R_{10}$ are -$OR_a$, (vi) compounds of formula Vf wherein one or two groups selected from of $R_1$, $R_3$, $R_5$, $R_6$, $R_7$, $R_8$ and $R_9$ are -$OR_a$, wherein $R_a$ is H or (C1-6)alkyl.

More preferred are compounds of formulas Va, Vb, Vd, Ve, Ve, Vf, wherein (i) $R_3$ and $R_6$ are - $OR_a$ or (ii) $R_5$ and $R_6$ are -$OR_a$, wherein $R_a$ is H or (C1-6)alkyl, or (iii) $R_3$ and $R_7$ are -$OR_a$, or (iv) $R_1$ and $R_3$ are -$OR_a$, or (v) $R_1$ and $R_5$ are -$OR_a$, or (vi) $R_1$ and $R_6$ are -$OR_a$, or (vii) $R_1$ and $R_7$ are -$OR_a$, or (viii) $R_6$ and $R_7$ are -$OR_a$, or (ix) $R_3$ and $R_5$ are -$OR_a$, or (x) $R_5$ and $R_7$ are -$OR_a$, wherein $R_a$ is H or (C1-6)alkyl.

In a third specific embodiment compounds are included, wherein (i) $R_8$, $R_9$, $R_{10}$, and one group selected from $R_3$, $R_5$, $R_6$, $R_7$, $R_{11}$ are -$OR_a$, or (ii) $R_8$, $R_9$, $R_{11}$, and one group selected from $R_3$, $R_5$, $R_6$, $R_7$, $R_{10}$ are -$OR_a$, or (iii) $R_9$, $R_{10}$, $R_{11}$, and one group selected from $R_3$, $R_5$, $R_6$, $R_7$, $R_8$ is - $OR_a$, wherein $R_a$ is H or C(1-6)alkyl.

In a fourth specific embodiment compounds are included, wherein $R_8$, $R_9$, $R_{10}$, and $R_{11}$ are -$OR_a$,

Thus compounds of the invention also include compounds of formula VIIa, VIIb, VIIc and all pharmaceutically acceptable salts or stereoisomers thereof,

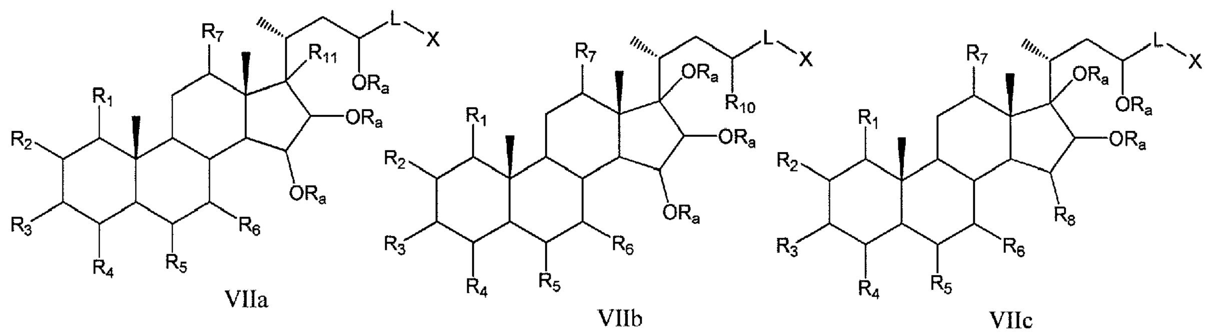

VIIa VIIb VIIc

wherein

| | |
|---|---|
| $R_2$, $R_4$, | are independently of each other H or hal, preferably H, |
| $R_1$, $R_3$, $R_5$, $R_6$, $R_7$, $R_8$, $R_9$, $R_{10}$, $R_{11}$, | are independently of each other H, hal, -$OR_a$, -$SR_a$, -$NR_aR_b$, - $COOR_a$, -$CONR_aR_b$, oxo, thio wherein $R_a$ and $R_b$ are independently of each other H or (C1-6)alkyl; |

| | |
|---|---|
| $R_1$, $R_3$, $R_5$, $R_6$, $R_7$ | are independently of each other H, hal, $-OR_a$, $-SR_a$, $-NR_aR_b$, $-COOR_a$, $-CONR_aR_b$, oxo, thio, preferably H, oxo, $-OR_a$, $-NR_aR_b$, wherein $R_a$ and $R_b$ are independently of each other H or (C1-6)alkyl; |
| L | is a covalent bond or a straight-chain or branched C(1-12)alkyl, which is unsubstituted and wherein one or more of the non-adjacent $CH_2$ groups may independently be replaced by a group selected from -O-, -CO-, - CO-O, preferably L is a straight-chain or branched C(1-12)alkyl; |
| X | is H, $-OR_a$, $-COOR_c$, $-CONR_aR_c$, wherein $R_a$ is H or (C1-6)alkyl and $R_c$ is -H, -(C1-6)alkyl, $-NH-(CH_2)_n-CO_2R_a$ or $-NH-(CH_2)_p-SO_3R_a$, wherein n, p is 1, 2, or 3 and $R_a$ is -H or -(C1-6)alkyl, and |
| $R_a$ | is H or C(1-6)alkyl, |
| m | is 0 to 5. |

In a fourth specific embodiment compounds are included, wherein $R_8$, $R_9$, $R_{10}$, and $R_{11}$ are $-OR_a$,

The term "alkyl" refers to a straight chain or branched carbon chain comprising the specified number of carbon atoms. Examples of alkyl groups include methyl, ethyl, n-propyl, isopropyl, n-butyl, 1-methylpropyl, 2-methylpropyl, tert-butyl, n-pentyl, 1-methylbutyl, 2-methylbutyl, 3-methylbutyl, 1,2-dimethylpropyl, 1,1-dimethylpropyl, 2,2-dimethylpropyl, n-hexyl, 1-methylpentyl, 2-methylpentyl, 3-methylpentyl, 4-methylpentyl, 1-ethylbutyl, 2-ethylbutyl, 3-ethylbutyl, 1,1-dimethyl butyl, 1,2-dimethylbutyl, 1,3-dimethylbutyl, 2,2-dimethylbutyl, 2,3-dimethylbutyl, 3,3-dimethyl butyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, 4-ethylpentyl, 1-propylbutyl, 2-propylbutyl, 3-propylbutyl, 1,1-dimethylpentyl, 1,2-dimethylpentyl, 1,3-dimethylpentyl, 1,4-dimethylpentyl, 2,2-dimethylpentyl, 2,3-dimethylpentyl. 2,4-dimethylpentyl, 3,3-dimethylpentyl, 3,4-dimethylpentyl, 4,4-dimethylpentyl, 1-methyl-1-ethylbutyl, 1-methyl-2-ethylbutyl, 2-methyl-2-ethylbutyl, 1-ethyl-2-methylbutyl, 1-ethyl-3-methylbutyl, 1,1-diethylpropyl, n-octyl, n-nonyl, n-decyl and the like.

Thus the term "C(1-6)alkyl" as used herein refers to branched or straight alkyl groups and includes "linear C(1-6)alkyl", such as methyl, ethyl, n-propyl, n-butyl, n-pentyl and n-hexyl, and "branched C(3-6)alkyl", such as isopropyl, isobutyl, tert-butyl, 2-pentyl, 3-pentyl, 2-methylbutyl, 3-methylbutyl, 3-methyl-2-butyl, 2,2-dimethyl-propyl, 2-hexyl, 3-hexyl, 3-methyl-2-pentyl, 3-methyl-3-pentyl, 2,2-dimethyl-butyl, 3,3-dimethyl-butyl, 2,3-dimethyl-2-butyl, 2,3-dimethyl-3-butyl and 3,3-dimethyl-2-butyl.

The term "halogen" (or "hal") includes fluoro, chloro, bromo and iodo, preferably chloro and bromo, more preferably bromo.

The term "oxo" refers to an oxygen atom connected by a double bond (=O).

The term "thio" refers to a sulfur atom connected by a double bond (=S).

Index "m" is 1, 2, 3, 4, or 5.

It is understood that if one or more of the above defined terms occur more than once in a formula (e.g. $R_a$, $R_b$, $R_c$), each term is defined independently of the other.

The compounds of the invention may contain one chiral plane, one or more asymmetric or chiral centers and can exist in different stereoisomeric forms, such as racemates and racemic mixtures, optically pure enantiomers, enantiomeric mixtures, optically pure diastereomers and diastereomeric mixtures. All stereoisomeric forms of the intermediates and compounds of the present invention as well as mixtures thereof, including racemic and diastereomeric mixtures, which possess properties useful in the treatment of the conditions discussed herein, form a part of the present invention.

The compounds of the invention may be separated into their individual enantiomers and diastereoisomers by standard techniques known in the art, such as e.g. fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration. Enantiomers and diastereomers may be separated by use of a chiral HPLC column and by converting the enantiomeric mixture into a diastereomeric mixture by reaction with an appropriate optically active compound (e.g., chiral auxiliary such as a chiral alcohol or Mosher's acid chloride), separating the diastereoisomers and converting (e.g., hydrolyzing) the individual diastereoisomers to the corresponding pure enantiomers. Alternatively, any stereoisomer of a compound of the invention may be obtained by stereospecific synthesis using optically pure starting materials or reagents of known absolute configuration.

Such ennatiomeric or diastereomeric separation or stereospecific synthesis may be particularly desirable if enantiomers or diastereomers differ in biological activity.

The present invention is meant to comprehend all such isomeric forms of the compounds of the invention, including the E and Z geometric isomers of double bonds and mixtures thereof. A number of the compounds of the present invention and intermediates therefore exhibit tautomerism and therefore may exist in different tautomeric forms under certain conditions. The term "tautomer" or "tautomeric form" refers to structural isomers of equal energies (in

50:50 mixtures) or different energies which are interconvertible via a low energy barrier. For example, proton tautomers include interconversions via migration of a proton, such as keto-enol and imine-enamine isomerizations. Valence tautomers include interconversions by reorganization of some of the bonding electrons. All such tautomeric forms are within the scope of the invention. The depiction of any particular tautomeric form in any of the structural formulas herein is not intended to be limiting with respect to that form; but is meant to be representative of the entire tautomeric set.

It is further understood that the compounds of the present invention include hydrates, solvates, polymorphs, crystalline, hydrated crystalline and amorphous forms of the compounds of the present invention, and pharmaceutically acceptable salts thereof.

For example, the compounds of the present invention and intermediates may exist in unsolvated as well as solvated forms with solvents such as water, ethanol, isopropanol and the like, and both solvated and unsolvated forms are included within the scope of the invention. Solvates for use in the methods aspect of the invention should be with pharmaceutically acceptable solvents.

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, lithium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, TEA, trimethylamine, tripropylamine, tromethamine, and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic acid, trifluoroacetic acid, and the like. Particularly preferred are citric, fumaric, hydrobromic, hydrochloric, maleic, phosphoric, sulfuric, and tartaric acids. It will be understood that, as used herein, references to the compounds of the invention are meant to also include the pharmaceutically acceptable salts, such as the hydrochloride salt.

In a further aspect the present invention also relates to methods of production of the compounds of the invention, which are able to overcome the disadvantages (e.g. poor yields) associated with the known prior art method.

The compounds of the invention can be obtained through standard synthetic methods as described in the Examples. Alternatively the compounds can be obtained using biocatalytic methods, for example using a suitable microbial strain or enzymes known to be able to perform oxidations, such as e.g. Cunninghamella blakesleeana, Streptomyces platensis, Absidia corymbifera, Mortierella isabellina and Beauveria bassiana, Rhizopus sp., Rhizopus arrhizus or, alternatively, the heme containing enzymes Cytochrome P450 monooxygenases.

The compounds of the invention are useful as effective ligands or modulators of the ROR receptor and in particular of the ROR gamma receptor. They are therefore useful for the treatment and/or prevention of disorders responsive to the modulation of the ROR gamma receptor, such as diabetes and diabetes-related disorders, and in particular type II diabetes.

Thus, the present invention also relates to the use of at least one compound of the invention as effective ligands or modulators of the ROR receptor and in particular of the ROR gamma receptor.

In particular, the present invention also relates to the use of at least one compound of the invention for the treatment or prevention, suppression or amelioration of a disease mediated by the ROR gamma receptor in a subject in need thereof.

More specifically, the present invention relates to the use of a therapeutically effective amount of at least one ROR gamma receptor ligand of formula I to VII, and any pharmaceutically acceptable salt or stereoisomer thereof, for the treatment or prevention, or suppression of a disease mediated by the ROR gamma receptor in a subject in need thereof, wherein the disease is selected from the group consisting of diabetes and diabetes-related disorders, in particular type II diabetes.

More specifically, the present invention relates to the use of a therapeutically effective amount of at least one compound of formula I to VII, and any pharmaceutically acceptable salt or stereoisomer thereof, for the treatment or prevention, or suppression of diabetes and/or diabetes-related disorders, in particular type II diabetes.

In some embodiments the compounds of the invention may be used singly, in other embodiments, the compounds of the invention may be used in combination with other compounds of the invention or in combination with other therapeutically active agents. Examples of other therapeutically active ingredients (for combined administration with one

ro more compounds of the invention) that may be useful for the treatment and/or prevention and/or amelioriation of the diseases or conditions for which the compounds of the invention are useful, i.e. diabetes and/or diabetes-related disorders, in particular type II diabetes, include, but are not limited to anti-diabetic agents, lipid-lowering agents, anti-hypertensive agents, and anti-obesity agents, such as the following:

(a) Anti-diabetic agents, for example, (1) glitazones (e.g., ciglitazone, darglitazone, englitazone, isaglitazon, pioglitazone, rosiglitazone, troglitazone, tularik, BRL49653, CLX-0921, 5-BTZD), and PPAR ligands such as GW-0207, LG-100641 and LY-300512; (2) biguanides such as buformin, metformin and phenformin; (3) protein tyrosine phosphatase-1B (PTP-1B) inhibitors; (4) sulfonylureas such as acetohexamide, chlorpropamide, diabinese, glibenclamide, glipizide, glyburide, glimepiride, gliclazide, glipentide, gliquidone, glisolamide, tolazamide and tolbutamide; (5) meglitinides such as repaglinide, nateglinide, and the like; (6) $\alpha$-glucosidase inhibitors such as acarbose, adiposine, camiglibose, emiglitate, miglitol, voglibose, pradimicin-Q, salbostatin, CKD-711, MDL-25,637, MDL-73,945, and MOR14; (7) $\alpha$-amylase inhibitors such as tendamistat, trestatin, and Al-3688; (8) insulin secretagogues such as linogliride, A-4166 and the like; (9) fatty acid oxidation inhibitors such as clomoxir, and etomoxir; (10) $\alpha$-2 antagonists such as midaglizole, isaglidole, deriglidole, idazoxan, earoxan, and fluparoxan; (11) insulin and insulin mimetics such as biota, LP-100, novarapid, insulin detemir, insulin lispro, insulin glargine, insulin zinc suspension (lente and ultralente), Lys-Pro insulin, GLP-1 (73-7) (insulintropin), and GLP-1 (7-36)-$NH_2$; (12) non-thiazolidinediones such as JT-501, farglitazar (GW-2570/GI-262579), and muraglitazar; PPAR antagonists, such as muraglitazar, and the compounds disclosed in US 6,414,002; (13) PPAR dual agonists such as MK-0767/KRP-297, CLX-0940, GW-1536, GW-1929, GW-2433, L-796449, LR-90, and SB219994; (14) other insulin sensitizers; (15) VPAC2 receptor agonists; (16) glucokinase activators; and (17) DPP-4 inhibitors, such as sitagliptin (Januvia™), isoleucine thiazolidide (P32/98); NVP-DPP-728; vildagliptin (LAF 237); P93/01; denagliptin (GSK 823093), SYR322, RO 0730699, TA-6666, and saxagliptin (BMS 477118).

(b) lipid lowering agents, for example, (1) bile acid sequestrants such as cholestyramine, colesevelam, colestipol, dialkylaminoalkyl derivatives of a cross-linked dextran, Colestid(R), LoCholest(R), and Questran(R), and the like; (2) HMG-CoA reductase inhibitors such as atorvastatin, itavastatin, fluvastatin, lovastatin, pitavastatin, pravastatin, rivastatin, rosuvastatin, and simvastatin, ZD-4522, and the like; (3) HMG-CoA synthase inhibitors; (4) cholesterol absorption inhibitors such as stanol esters, beta -sitosterol, sterol glycosides such as tiqueside, and azetidinones like ezetimibe; (5) acyl coenzyme A-cholesterol acyl-transferase (ACAT) inhibitors such as avasimibe, eflucimibe, KY505, and SMP797, and the like; (6) CETP inhibitors such as JTT705, torcetrapib, CP532632, BAY63-2149, SC591, and SC795, and the like; (7) squalene synthase inhibitors; (8) antioxidants such as probucol; (9) PPAR-a agoists such as beclofibrate, benzafibrate, ciprofibrate, clofibrate, etofibrate, fenofibrate, gemcabene, gemfibrozil, and other fibric acid derivatives, e.g., GW7647, BM170744, LY518674, Atromid(R), Lopid(R), and Tricor(R), and compounds described in WO 97/36579, and the like; (10) FXR receptor modulators such as GW4064, SR103912, and the like; (11) LXR receptor ligands such as GW3965, T9013137, and XTCO179628, and the like; (12) lipoprotein synthesis inhibitors such as niacin; (13) renin/angiotensin system inhibitors; (14) PPAR-d partial agonists; (15) bile acid reabsorption inhibitors such as BARI1453, SC435, PHA384640, S8921, AZD7706, and the like; (16) PPAR-d agonists such as GW501516, GW590735, and compounds described in WO97/28149, and the like; (17) triglyceride synthesis inhibitors, (18) microsomal triglyceride transport (MTTP) inhibitors such as inplitapide, LAB687, and CP346086; (19) transcription modulators, (20) squalene epoxidase inhibitors; (21) low-density lipoprotein (LDL) receptor inducers; (22) platelet aggregation inhibitors; (23) 5-LO or FLAP inhibitors; and (24) niacin receptor agonists; and

(c) anti-hypertensive agents, for example, (1) diuretics such as thiazides including chlorthalidone, chlorothiazide, dichlorphenamide, hydroflumethiazide, indapamide and hydrochlorothiazide; loop diuretics such as bumetanide, ethacrynic acid, furosemide, and torsemide; potassium sparing agents such as amiloride, triamterene; aldosterone antagonists such as spironolactone, and epirenone, and the like; (2) beta -adrenergic blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, propanolol, sotalol, tertatolol, tilisolol, and timolol, and the like; (3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodipine, nisoldipine, nitrendipine, manidipine, pranidipine, and verapamil, and the like; (4) angiotensin converting enzyme (ACE) inhibitors such as benazepril, captopril, cilazapril, delapril, enalapril, fosinopril, imidapril, lisinopril, moexipril, quinapril, quinaprilat, ramipril, perindopril, perindropril, quanipril, spirapril, tenocapril, trandolapril, and zofenopril, and the like; (5) neutral endopeptidase inhibitors such as omapatrilat, cadoxatril, ecadotril, fosidotril, sampatrilat, AVE7688, ER4030, and the like; (6) endothelin antagonists such as bosentan, tezosentan, A308165, and YM62899, and the like; (7) vasodilators such as hydralazine, clonidine, minoxidil, and

nicotinyl alcohol; (8) angiotensin II receptor antagonists such as candesartan, eprosartan, irbesartan, losartan, losartan and hydrochlorothiazide, pratosartan, tasosartan, telmisartan, valsartan, EXP-3137, FI6828K, and RNH6270, and the like; (9) a/ beta -adrenergic blockers such as nipradilol, arotinolol, and amosulalol; (10) al blockers such as terazosin, urapidil, prazosin, bunazosin, trimazosin, doxazosin, naftopidil, indoramin, WHIP164, and XEN010; (11) a2 agonists such as lofexidine, tiamenidine, moxonidine, rilmenidine, and guanobenz; (12) aldosterone inhibitors; and

(d) anti-obesity agents, such as (1) growth hormone secretagogues, growth hormone secretagogue receptor agonists/antagonists, such as NN703, hexarelin, MK-0677, SM-130686, CP-424,391, L-692,429, and L-163,255; (2) protein tyrosine phosphatase-1B (PTP-1B) inhibitors; (3) cannabinoid receptor ligands, such as cannabinoid $CB_1$ receptor antagonists or inverse agonists, such as rimonabant (Sanofi Synthelabo), AMT-251, and SR-14778 and SR 141716A (Sanofi Synthelabo), SLV-319 (Solvay), BAY 65-2520 (Bayer); (4) anti-obesity serotonergic agents, such as fenfluramine, dexfenfluramine, phentermine, and sibutramine; (5) beta 3-adrenoreceptor agonists, such as AD9677/TAK677 (Dainippon/Takeda), CL-316,243, SB 418790, BRL-37344, L-796568, BMS-196085, BRL-35135A, CGP12177A, BTA-243, Trecadrine, Zeneca D7114, SR 59119A; (6) pancreatic lipase inhibitors, such as orlistat (Xenical(R)), Triton WR1339, RHC80267, lipstatin, tetrahydrolipstatin, teasaponin, diethylumbelliferyl phosphate; (7) neuropeptide Y1 antagonists, such as BIBP3226, J-115814, BIBO 3304, LY-357897, CP-671906, GI-264879A; (8) neuropeptide Y5 antagonists, such as GW-569180A, GW-594884A, GW-587081X, GW-548118X, FR226928, FR 240662, FR252384, 1229U91, GI-264879A, CGP71683A, LY-377897, PD-160170, SR-120562A, SR-120819A and JCF-104; (9) melanin-concentrating hormone (MCH) receptor antagonists, such as those disclosed in WO 01/21577 and WO 01/21169; (10) melanin-concentrating hormone 1 receptor (MCH1R) antagonists, such as T-226296 (Takeda); (11) melanin-concentrating hormone 2 receptor (MCH2R) agonist/antagonists; (12) orexin-1 receptor antagonists, such as SB-334867-A, and those disclosed in WO 01/96302, WO 01/68609, WO 02/51232, and WO 02/51838; (13) serotonin reuptake inhibitors such as fluoxetine, paroxetine, and sertraline; (14) melanocortin agonists, such as Melanotan II, CHIR86036 (Chiron), ME-10142, and ME-10145 (Melacure), CHIR86036 (Chiron); PT-141, and PT-14 (Palatin); (15) other MC4R (melanocortin 4 receptor) agonists; (16) 5HT-2 agonists; (17) 5HT2C (serotonin receptor 2C) agonists, such as BVT933, DPCA37215, WAY 161503, R-1065; (18) galanin antagonists; (19) CCK agonists; (20) CCK-1 agonists (cholecystokinin-A) agonists, such as AR-R 15849, GI 181771, JMV-180, A-71378, A-71623 and SR146131; (21) GLP-1 agonists; (22) corticotropin-releasing hormone agonists; (23) histamine receptor -3 (H3) modulators; (24) histamine receptor -3 (H3) antagonists/inverse agonists, such as hioperamide, 3-(1H-imidazol-4-yl)propyl N-(4-pentenyl)carbamate, clobenpropit, iodophenpropit, imoproxifan, GT2394 (Gliatech), and O-[3-(1H-imidazol-4-yl)propanol]-carbamates (Kiec-Kononowicz, K. et al., Pharmazie, 55:349-55 (2000)), piperidine-containing histamine H3-receptor antagonists (Lazewska, D. et al., Pharmazie, 56:927-32 (2001), benzophenone derivatives and related compounds (Sasse, A. et al., Arch. Pharm.(Weinheim) 334:45-52 (2001)), substituted N-phenylcarbamates (Reidemeister, S. et al., Pharmazie, 55:83-6 (2000)), and proxifan derivatives (Sasse, A. et al., J. Med. Chem. 43:3335-43 (2000)); (25) beta-hydroxy steroid dehydrogenase-1 inhibitors (beta -HSD-1); 26) PDE (phosphodiesterase) inhibitors, such as theophylline, pentoxifylline, zaprinast, sildenafil, amrinone, milrinone, cilostamide, rolipram, and cilomilast; (27) phosphodiesterase-3B (PDE3B) inhibitors; (28) NE (norepinephrine) transport inhibitors, such as GW 320659, despiramine, talsupram, and nomifensine; (29) ghrelin receptor antagonists; (30) leptin, including recombinant human leptin (PEG-OB, Hoffman La Roche) and recombinant methionyl human leptin (Amgen); (31) leptin derivatives; (32) other BRS3 (bombesin receptor subtype 3) agonists such as [D-Phe6,beta-Ala11,Phe13,Nle14]Bn(6-14) and [D-Phe6,Phe13]Bn(6-13)propylamide; (33) CNTF (Ciliary neurotrophic factors), such as GI-181771 (Glaxo-SmithKline), SR146131 (Sanofi Synthelabo), butabindide, PD170,292, and PD 149164 (Pfizer); (34) CNTF derivatives, such as axokine (Regeneron); (35) monoamine reuptake inhibitors, such as sibutramine; (36) UCP-1 (uncoupling protein-1), 2, or 3 activators, such as phytanic acid, 4-[(E)-2-(5,6,7,8-tetrahydro-5,5,8,8-tetramethyl-2-napthalenyl)-1-propenyl]benzoic acid (TTNPB), retinoic acid; (37) thyroid hormone beta agonists, such as KB-2611 (KaroBioBMS); (38) FAS (fatty acid synthase) inhibitors, such as Cerulenin and C75; (39) DGAT1 (diacylglycerol acyltransferase 1) inhibitors; (40) DGAT2 (diacylglycerol acyltransferase 2) inhibitors; (41) ACC2 (acetyl-CoA carboxylase-2) inhibitors; (42) glucocorticoid antagonists; (43) acyl-estrogens; (44) dipeptidyl peptidase IV (DP-IV) inhibitors, such as isoleucine thiazolidide, valine pyrrolidide, NVP-DPP728, LAF237, P93/01, TSL 225, TMC-2A/2B/2C, FE 999011, P9310/K364, VIP 0177, SDZ 274-444 and sitagliptin; (46) dicarboxylate transporter inhibitors; (47) glucose transporter inhibitors; (48) phosphate transporter inhibitors; (49) Metformin (Glucophage(R)); and (50) Topiramate (Topimax(R)); and (50) peptide YY, PYY 3-36, peptide YY analogs, derivatives, and fragments such as BIM-43073D, BIM-43004C (Olitvak, D.A. et al., Dig. Dis. Sci. 44(3):643-48 (1999)); (51) Neuropeptide Y2 (NPY2) receptor agonists such NPY3-36, N acetyl [Leu(28,31)] NPY 24-36, TASP-V, and cyclo-(28/32)-Ac-[Lys28-Glu32]-(25-36)-pNPY; (52) Neuropeptide Y4 (NPY4) agonists such as pancreatic peptide (PP) as described in Batterham et al., J. Clin. Endocrinol. Metab. 88:3989-3992 (2003), and other Y4 agonists such as 1229U91; (54) cyclooxygenase-2 inhibitors such as etoricoxib, celecoxib, valdecoxib, parecoxib, lumiracoxib,

BMS347070, tiracoxib or JTE522, ABT963, CS502 and GW406381, and pharmaceutically acceptable salts thereof; (55) Neuropeptide Y1 (NPY1) antagonists such as BIBP3226, J-115814, BIBO 3304, LY-357897, CP-671906, GI-264879A; (56) Opioid antagonists such as nalmefene (Revex (R)), 3-methoxynaltrexone, naloxone, naltrexone; (57) 11 beta HSD-1 (11-beta hydroxy steroid dehydrogenase type 1) inhibitors such as BVT 3498, BVT 2733; and other compounds such as aminorex; amphechloral; amphetamine; benzphetamine; chlorphentermine; clobenzorex; cloforex; clominorex; clortermine; cyclexedrine; dextroamphetamine; diphemethoxidine, N-ethylamphetamine; fenbutrazate; fenisorex; fenproporex; fludorex; fluminorex; furfurylmethylamphetamine; levamfetamine; levophacetoperane; mefenorex; metamfepramone; methamphetamine; norpseudoephedrine; pentorex; phendimetrazine; phenmetrazine; picilorex; phytopharm 57; zonisamide, neuromedin U and analogs or derivatives thereof, oxyntomodulin and analogs or derivatives thereof, Neurokinin-1 receptor antagonists (NK-1 antagonists); and Qnexa.

The compounds of the invention may be used in form of a pharmaceutical composition. Thus, in another aspect, the present invention provides a pharmaceutical composition comprising at least one compound of the invention (or a pharmaceutically acceptable salt thereof) and a pharmaceutically acceptable carrier and optionally at least one further therapeutically active agent as defined above. The term "pharmaceutically acceptable salts" refers to salts prepared from pharmaceutically acceptable non-toxic bases or acids including inorganic bases or acids and organic bases or acids as defined hereinabove.

Conveniently, the compounds of the invention may be combined in intimate admixture with a pharmaceutical carrier according to conventional pharmaceutical techniques. The carrier may take a wide variety of forms depending on the form of preparation desired for administration (as defined hereinafter).

The compounds of the invention are ROR (gamma) receptor ligands and have been shown to be useful in the treatment, control or prevention of diseases, disorders or conditions responsive to the modulation of the ROR (gamma) receptor, e.g. diabetes and diabetes-related disorders, in particular type II diabetes.

Therefore, the present invention provides in a further aspect a method for the treatment or prevention of disorders, diseases or conditions responsive to the modulation of the ROR gamma receptor in a subject in need thereof, which comprises administering to the subject a therapeutically or prophylactically effective amount of at least one compound of the invention, or a pharmaceutically acceptable salt thereof, optionally in combination with at least one further therapeutically active agent as defined above.

More specifically, the present invention provides a method for the treatment or prevention of diabetes and diabetes-related disorders in a subject in need thereof which comprises administering to said subject a therapeutically or prophylactically effective amount of at least one ROR gamma receptor ligand of the present invention, optionally in combination with at least one further therapeutically active agent as defined above.

In particular, the present invention provides a method for the treatment or prevention of type II diabetes in a subject in need thereof which comprises administering to the subject a therapeutically or prophylactically effective amount of at least one compound of the invention, or a pharmaceutically acceptable salt thereof, optionally in combination with at least one further therapeutically active agent as defined above.

The terms "administration of" or "administering" a compound of the invention should be understood to mean providing a compound of the invention (or a prodrug of a compound of the invention) or a pharmaceutical composition thereof to a subject in need of treatment. The administration of the compounds of the present invention in order to practice the present methods of therapy is carried out by administering a therapeutically or prophylactically effective amount of the compound (or composition) of the invention and optionally a therapeutically effective amount of at least one further therapeutically active compound to a subject in need of such treatment or prophylaxis.

The term "therapeutically effective amount" as used herein means the amount of a compound of the invention that will elicit the desired biological or medical response in a tissue, system, or subject, which includes alleviation of the symptoms of the disorder being treated. The term "prophylactically effective amount" as used herein means the amount of a compound of the invention that will elicit the desired biological or medical response in a tissue, system, or subject to prevent the onset of the disorder in subjects as risk for the disorder. The therapeutically or prophylactically effective amount, or dosage, of a specific compound of the invention is determined by the physician, but depends on factors such as the exact disease to be treated, the severity of the disease and other diseases or conditions from which the patient suffers, the chosen route of administration, other drugs and treatments which the patient may concomitantly require, the patients age and constitution and other factors in the physician's judgement.

Any suitable route of administration may be employed for providing a subject, especially a human with a therapeutically effective dosage of a compound of the present invention. For example, oral, rectal, topical, parenteral (e.g. subcutaneous or intravenous infusion, and subcutaneous or intravenous injection), ocular, pulmonary, nasal, and the like may be employed. Dosage forms include tablets, troches, dispersions, suspensions, solutions, capsules, creams, ointments, aerosols, and the like. Preferably compounds of the invention are administered orally or parenterally.

The effective dosage of active ingredient employed may vary depending on the particular compound of choice, the mode of administration, the condition being treated and the severity of the condition being treated. Such dosage

may be ascertained readily by a person skilled in the art.

Typically a compound or the invention (or combinations thereof) are administered at a daily dosage of from about 0.001 mg to about 50 mgs per kilogram of subject body weight, preferably given in a single dose or in divided doses two to six times a day, or in sustained release form. For example, in the case of a 70 kg adult human, the total daily dose will generally be from about 0.07 mgs to about 3500 mgs. This dosage regimen may be adjusted to provide the optimal therapeutic response. In case of oral administration, a suitable dosage range is, e.g. from about 0.01 mg to about 1500 mg of one or more compounds of the invention per day, preferably from about 0.1 mg to about 600 mg per day, more preferably from about 0.1 mg to about 100 mg per day. For oral administration, the compositions are preferably provided in the form of tablets containing from 0.01 to 1,000 mg, preferably 0.01, 0.05, 0.1, 0.5, 1, 2.5, 5, 10, 15, 20, 25, 30, 40, 50, 100, 250, 500, 600, 750, 1000, 1250 or 1500 mgs of the active ingredient for the symptomatic adjustment of the dosage to the patient to be treated. In case of intranasal administration, a suitable dosage range is e.g. a formulation comprising 0.001-10 percent by weight solutions or suspensions of one or more compounds of the invention may be used. In case of intravenous administration, a suitable dosage range is from about 0.001 mg to about 50 mg, preferably from 0.01 mg to about 50 mg, more preferably 0.1 mg to 10 mg, of one or more compounds of the invention per kg of body weight per day.

The above dosage regimen may be adjusted to provide the optimal therapeutic response. It may be necessary to use dosages outside these limits in some cases. The exact amount of prophylactic or therapeutic dosage of one or more compounds of the invention will, of course, vary depending on the particular compound or compounds of choice, the mode of administration, the condition being treated and the severity of the condition being treated. It will also vary according to the age, weight and response of the individual patient. Such dosage may be ascertained readily by a person skilled in the art.

As indicated above, the present invention also contemplates the use of compounds of the present invention in combination with at least one further therapeutically active compound. Such further therapeutically active compound may be administered, by a route and in an amount commonly used therefore, and may be administered separately or combined (in single or separate pharmaceutical compositions), simultaneously or sequentially with one or more compounds of the invention. In case of simultaneous use, a pharmaceutical composition comprising both the at least one compound of the invention and the at least one additional therapeutically active agent may be used. Accordingly, the present invention also refers to administration of a single pharmaceutical dosage formulation comprising at least one ROR gamma receptor ligand in combination with at least one further therapeutically active agent, as well as administration of each active agent in its own separate pharmaceutical dosage formulation. Where separate dosage formulations are used, the individual components of the composition can be administered at essentially the same time, i.e., concurrently, or sequentially, i.e. prior to or subsequent to the administration of the other component of the composition. It is understood that the present invention includes all such regimes of simultaneous or sequential treatment, and the terms "administration" and "administering" are to be interpreted accordingly.

Various organic or inorganic carrier materials conventionally used as materials for pharmaceutical preparations may be used as pharmaceutically acceptable carrier for the compounds of the invention and the route of administration chosen. They are blended as excipient, lubricant, binder or disintegrant for solid preparations; and solvent, solubilizing agent, suspending agent, isotonicity agent, buffer, soothing agent and the like for liquid preparations. Where necessary, an additive for pharmaceutical preparations such as preservative, antioxidant, colorant, sweetening agent and the like can be used.

Typical examples of an excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate, magnesium aluminate metasilicate and the like. Typical examples of a lubricant include magnesium stearate, calcium stearate, talc, colloidal silica and the like. Typical examples of a binder include pregelatinized starch, saccharose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropyl methylcellulose, polyvinylpyrrolidone and the like. Typical examples of a disintegrant include lactose, sucrose, starch, carboxymethylcellulose, calcium carboxymethylcellulose, sodium croscarmellose, sodium carboxymethyl starch, light anhydrous silicic acid, low-substituted hydroxypropylcellulose and the like. Typical examples of a solvent include water for injection, physiological saline, Ringer's solution, alcohol, propylene glycol, polyethylene glycol, sesame oil, corn oil, olive oil, cottonseed oil and the like. Typical examples of a solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like. Typical examples of a suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil; and the like. Typical examples of an isotonicity agent include sodium chloride, glycerol, D-mannitol, D-sorbitol, glucose and

the like. Typical examples of a buffer include phosphate buffer, acetate buffer, carbonate buffer, citrate buffer and the like. Typical examples of a soothing agent include benzyl alcohol and the like. Typical examples of a preservative include p-oxybenzoates, chlorobutanol, benzyl alcohol, phenethyl alcohol, dehydroacetic acid, sorbic acid and the like. Typical examples of an antioxidant include sulfite, ascorbate and the like. Typical examples of a colorant include aqueous edible tar pigments (e.g., foodcolors such as Food Color Red Nos. 2 and 3, Food Color Yellow Nos. 4 and 5, Food Color Blue Nos. 1 and 2 etc.), water insoluble lake pigments (e.g., aluminum salt of the aforementioned aqueous edible tar pigment), natural pigments (e.g., beta carotene, chlorophyll, red iron oxide and yellow ferric oxide) and the like. Typical examples of a sweetening agent include saccharin sodium, dipotassium glycyrrhizinate, aspartame, stevia and the like.

Examples of a suitable dosage form for a compound of the present invention include oral preparations such as tablet (including sugar-coated tablet, film-coated tablet, sublingual tablet, orally disintegrating tablet), capsule (including soft capsule, microcapsule), granule, powder, troche, syrup, emulsion, suspension, film (e.g., mouth cavity disintegrating film) and the like; or parenteral preparations such as injections (e.g., subcutaneous injections, intravenous injections, intramuscular injections and intraperitoneal injections), external agents (e.g., preparations for nasal administration, transdermal preparations and ointments), suppositories (e.g., rectal suppositories and vaginal suppositories), pellets, drops, eye drops, pulmonary preparations (inhalations) and the like. In addition, these preparations may be sustained-release preparations (e.g., sustained-release microcapsule), such as an immediate release preparation or a sustained-release preparation.

The pharmaceutical compositions of the invention may be produced according to a method conventionally used in the field of pharmaceutical preparations. The content of the at least one compound of the invention in a pharmaceutical composition varies depending on the nature of the compound, the route of administration (and thus the nature of the pharmaceutical preparation), etc., and is e.g. from 1 to 90 wt %, preferably from 5 to 80 wt %.

In a further aspect the present invention also relates to a kit providing a pharmaceutical composition of the invention comprising at least one compound of the invention and optionally at least one further therapeutically active compound. In one embodiment, the kit, according to this invention, may comprise a single oral dosage formulation comprising both at least one ROR (gamma) receptor ligand and at least one further therapeutically active ingredient in one compartment of the kit. In another embodiment, the kit, according to this invention, may comprise at least two separate pharmaceutical compositions in separate compartments, which are a first unit dosage form comprising a prophylactically or therapeutically effective amount of at least one ROR (gamma) receptor ligand, or a pharmaceutically acceptable salt or ester thereof, and a pharmaceutically acceptable carrier or diluents in a first compartment, and a second unit dosage form comprising a prophylactically or therapeutically effective amount of at least one further active ingredient, or a pharmaceutically acceptable salt or ester thereof, and a pharmaceutically acceptable carrier or diluent in a second compartment.

The present invention is further exemplified by the following specific examples, which are intended to illustrate the invention and are not to be construed as limiting the scope of the invention in any manner. It is understood that a skilled person in the art can readily prepare additional compounds of the present invention based on the procedures described herein. Those skilled in the art will readily understand that known variations of the conditions and processes of the following preparative procedures can be used to prepare these compounds.

## Examples

### Materials and Methods

All temperatures are degrees Celsius unless otherwise noted. All non-aqueous reactions were performed in flame dried glassware under an atmosphere of argon / nitrogen unless stated otherwise.

All chemicals and solvents were purchased from ABCR, Acros, Aldrich, J.T. Baker, Fluka, Merck, TCI, or Lancaster and used as such with the following exceptions: THF and DMF were dried by passage over two 4" × 36" columns of anhydrous neutral A-2 alumina (Macherey und Nagel; activated over night at 300 °C under a flow of $N_2$) under an atmosphere of argon ($H_2O$ content < 30 ppm, *Karl-Fischer* titration). TLC was performed on Merck silica gel 60 F254 TLC aluminum plates and visualized with UV fluorescence quenching and *p*-Anysaldehyde-stain. Solid phase extractions were performed using Agilent SPE C-18 cartridges, (60 mL, 10 g C-18) or ordinary glass columns containing C-18 stationary phase. Elutions were performed applying 0.3-0.5 bar pressure of $N_2$. Concentrations under reduced pressure were performed by rotary evaporation at 37 °C at the appropriated pressure, unless otherwise noted. Column chromatographic purification was performed as flash chromatography with 0.3-0.5 bar pressure on silica gel (60 A, 40-64 μm). Distilled technical grade solvents were employed.

NMR data was recorded on a *VARIAN Mercury* 300 MHz, *Gemini* 300 MHz, *Bruker ARX* 300, *Bruker AV* 400, *Bruker DRX* 400 *Bruker AV* 600 spectrometer. Measurements were carried out at RT (*ca.* 22 °C) unless otherwise noted. Chemical shifts are given in ppm with the residual solvent signal as internal standard [chloroform at 7.26 and 77.23 ppm, $d_6$-DMSO at 2.50 and 39.52 ppm, $CD_3OD$ at 3.32 and 49.00 ppm, for $^1H$- and $^{13}C$, respectively]. Multiplicities are

abbreviated as follows: singlet (s), doublet (d), triplet (t), apparent triplet (at), doublet-doublet (dd), triplet-doublet (td), doublet-doublet-doublet (ddd), and multiplet (m), coupling constant(s) are given in Hz. $^{13}C$-NMR spectra were recorded with 1H-decoupling, multeplicities were deduced by phase sensitive HSQC experiments. Infrared spectra were recorded on a *Perkin Elmer RXI FT-IR* Spectrophotometer as thin films, absorptions are given in wavenumbers ($cm^{-1}$). Analytical HPLC-MS was performed using a MS detector, negative ionization method, gradient A = 3% B = 97% to A 97%, B = 3%, 40 min where A = water, B = acetonitrile + 0.01 % formic acid. Flow = 1 mL/min. column: C18, 5 mm length. Preparative HPLC was performed using: column: prepspher C18 acqua, 5$\mu$m, 150 x 30 mm, gradient A to B 10 to 30 % and/or A to B 20 to 40 % in 20 min., where A = water + 0.01% HCOOH, B = acetonitrile + 0.01 % HCOOH, flow 45 mL/min., detection: UV 205 nm. Mass spectrometric analyses were performed by the mass spectrometry service of the *Laboratorium für Organische Chemie* at *ETH Zürich* by L. Bertschi and O. Greter under direction of Dr. Zhang. ESI and HRMS measurements were carried out on a *Bruker Daltonics maxis* and *Varian IonSpec ESI FT-ICR* at 4.7 Tesla. EI measurements were carried out on a *Waters Micromass AutoSpec Ultima* at 70 eV.

The use of protecting groups for the amine and carboxylic acid functionalities to facilitate the desired reaction and minimize undesired reactions is well documented. Conditions required to remove protecting groups are found in standard textbooks such as Greene, T, and Wuts, P. G. M., Protective Groups in Organic Synthesis, John Wiley and Sons, Inc., New York, NY, 1991. CBZ and BOC are commonly used protecting groups in organic synthesis, and their removal conditions are known to those skilled in the art. For example, CBZ may be removed by catalytic hydrogenation in the presence of a noble metal or its oxide such as palladium on activated carbon in a protic solvent such as methanol or ethanol. In cases where catalytic hydrogenation is contraindicated due to the presence of other potentially reactive functionalities, removal of CBZ groups can also be achieved by treatment with a solution of hydrogen bromide in acetic acid or by treatment with a mixture of TFA and dimethylsulfide. Removal of BOC protecting groups is carried out with a strong acid, such as trifluoroacetic acid, hydrochloric acid, or hydrogen chloride gas, in a solvent such as methylene chloride, methanol, or ethyl acetate.

<u>Example 1: Isolation of 3α,7β,15α,(23R)-tetrahydroxy-5β-24-oxocholanyl-amino ethansulfonic acid.</u>

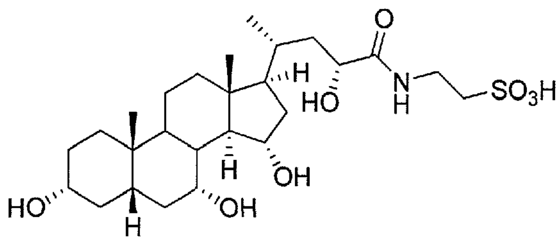

Foie gras obtained from duck (1.4 kg) was divided in 4 portions of 350 g each, which were separately suspended in ethanol (700 mL), refluxed for 3 h, and sonicated. Each batch was centrifuged and the supernatant was collected, the pellet was re-suspended in ethanol (600 mL), sonicated and centrifuged. The procedure of sonication/centrifugation was repeated using a mixture of ethanol water 1 : 1 (600 mL). All the extracts were combined, the solvents were evaporated under reduced pressure and the residue was re-suspended in water/methanol 1 : 1 (500 mL), sonicated and centrifuged. All the supernatants were collected, the solvents were evaporated under reduced pressure and the residue was suspended in water methanol 4 : 1 (60 mL) and filtered through a pad of celite. Evaporation of the solvents under reduced pressure gave a residue which was re-suspended in methanol water 1 : 4, and loaded into a SPE extraction column (60g of C-18 stationary phase, previously conditioned with $CHCl_3$/Methanol 1 : 1, methanol and distilled water). The elution was performed using distilled water (120 mL), water/methanol 4 : 1 (120 mL) water/methanol 3 : 1 (120 mL), water/methanol 2 : 1 (120 mL) and 1: 1 (120 mL), and finally pure methanol (120 mL). The fractions were collected and analyzed by LC-MS, showing significant presence in the water/methanol 2 : 1 and 1 : 1 fractions of tetrahydroxylated tauroconjugate bile acids (MW = 530.3), along with tri and di-hydroxylated (MW = 514.3 and 498.3, respectively). The fractions containing the tetrahydroxy bile acids (MW = 530.3) were further analyzed by HPLC-MS showing the presence three isomeric peaks, peak **A** (r.t. = 9.0 min, minor peak) peak **B,** (r.t. = 10.3 min., major peak) and peak **C** (r.t. = 10.7 min, traces). The major compound corresponding to peak **B** was partially purified by preparative HPLC of the SPE extracts, followed by fraction collection and LC-MS analysis. Purification of the fractions containing the molecular peak of 530.3 was effected by silica gel flash chromatography ($CHCl_3$/MeOH/$CH_3COOH$ 4:2:0.1, Rf = 0.39) yielding a pure tauroconjugate tetrahydroxy bile acid (3.5 mg, 0.00025 %), the structure of which was determined by mono and bidimentional NMR. The position and stereochemistry of the four hydroxyl groups (C3, C7, C15, C23) in the molecule was

determined by mono and bidimentional NMR experiments ($^{1}H$, $^{13}H$, COSY, HSQC, HMBC). Analysis: Colorless amorphous; $[\alpha]_D^{20} = +17.7$ (*c* = 0.14 in MeOH); *Rf* = 0.39 ($CHCl_3$/MeOH/HAc 4 : 2 : 1) $^{1}H$ NMR (600 MHz, $CD_3OD$): δ 4.08 (dd, *J* = 11.7, 2.0 Hz, 1H, H-23), 4.02 (dd, *J* = 5.6, 3.5 Hz, 1H, H-7), 3.98 (td, *J* = 9.2, 3.0 Hz, 1H, H-15), 3.70-3.60 (m, 2H, H-25), 3.42- 3.37 (m, 1H, H-3), 2.98 (t, *J* = 6.2 Hz, 2H, H-26), 2.19 (dd, *J* = 24.6, 13.1 Hz, 1H, H-4a), 2.08 (ddd, *J* = 14.8, 5.4, 4.3 Hz, 1H, H-6a), 2.04-1.97 (m, 1H, H-12a), 1.90-1.82 (m, 3H, H16a, H-9, H-1a), 1.74 (ddd, *J* = 14.0, 8.7, 3.0 Hz, 1H, H-16b), 1.70-1.65 (m, 2H, H-20, H-8), 1.65-1.56 (m, 3H, H-22a, H-4b, H-2a), 1.56-1.49 (m, 3H, H-14, H-11a, H-6b), 1.48-1.42 (m, 1H, H-17), 1.42-1.27 (m, 5H, H-22b, H12b, H11b, H5, H-2b), 1.01 (d, *J* = 6.4 Hz, 3H, H-21), 1.01-0.95 (m, 1H, H-1b), 0.93 (s, 3H, H-19), 0.76 (s, 3H, H-18); $^{13}C$ NMR (101 MHz, $CD_3OD$): δ 178.4 (s, C-24), 72.8 (d, C-15), 72.7 (d, C3), 70.4 (d, C-23), 68.9 (d, C-7), 59.5 (d, C-14), 56.3 (d, C-17), 51.4 (t, C-26), 45.0 (s, C-13), 42.9 (d, C-5), 41.8 (t, C-22), 41.7 (t, C-12), 41.2 (t, C-16), 40.5 (d, C-8), 40.0 (t, C-4), 36.5 (t, C-1), 36.1 (t, C-25), 35.9 (s, C-10), 35.4 (d, C-9), 34.1 (t, C-6), 33.0 (d, C-20), 31.2 (t, C-2), 23.3 (q, C-19), 21.8 (t, C-11), 18.2 (q, C-21), 13.6 (q, C-18); IR (film): ν = 3362, 2928, 2865, 1650, 1448, 1199, 1170, 1148 $cm^{-1}$; HRMS ($ES^-$): *m*/*z*: Calcd for $C_{26}H_{45}NO_8S$(M-H): 530.2793, found: 530.2792.

Example 2: Synthesis of 3α,7β, 15α, (23R)-tetrahydroxy-5β-24-oxocholanyl-amino ethansulfonic acid.

Method A (Schemes 1a and 1b)

Commercially available chenodeoxycholic acid was converted into the corresponding chenodeoxycholic acid methylester **1,** subjected to treatment with $ZnCl_2$ in acetone under reflux (Nonappa; Mitra U. Steroids, 2010, 506-512), to give the corresponding $\Delta^{8,14}$ olefin and protected at the OH functionality to obtain the corresponding MOM ether **2** (Scheme 1a). A double allylic oxidation converted the unsaturated derivative **2** into the allylic diol **3** (Osawa R. Bulletin of the Chemical Society of Japan, 1962 , 158-162), which in turn can be selectively oxidized using $CrO_3$ in the presence of pyridine to give **4** (Schroepfer and Parish, Chemistry and Physics of Lipids, 1980, 282-288). Lithium in liquid ammonia reduction of **4** afforded diol **5,** with the desired stereochemistry at the new 8, 14 and 15 stereocenters (Tohma M. et al Chem. Pharm. Bull. 1989, 2147-2152). Protection and installation of the 23 OH group using KHMDS and Davies oxaziridine (Fiander and Schneider; Bioorganic and Medicinal Chemistry Letters, 1996, 637-642) gave the protected tetraol 7, which after hydrolysis (step 2) afforded the free acid.

The obtained tetrahydoxylated bile acid was converted into the corresponding tauroconjugate compound **8a** (Pellicciari R. J. Med Chem., 1985, 239-242) in one step (scheme 1b).

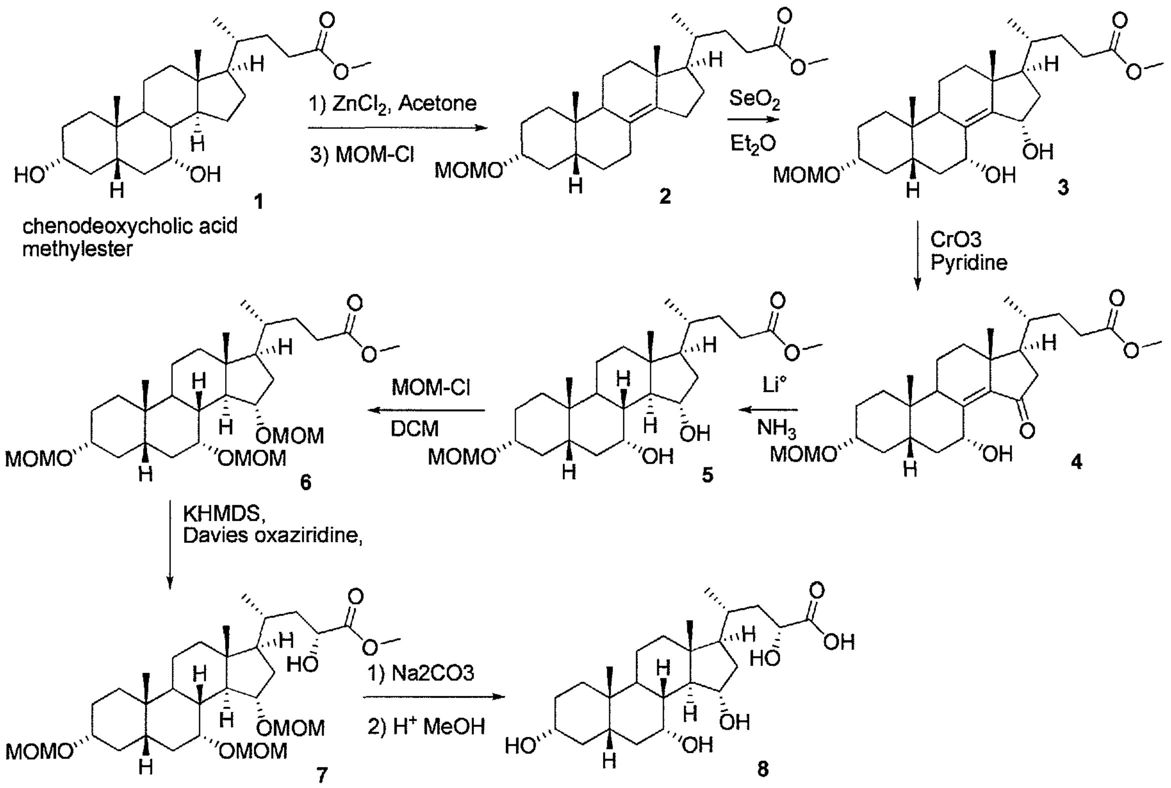


Scheme 1a

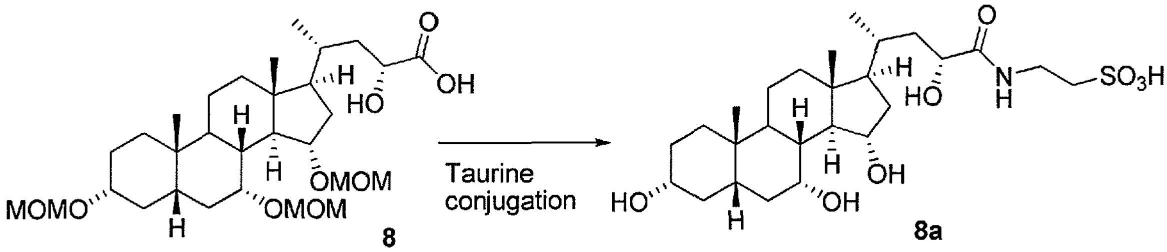


Scheme 1b

Method B (Scheme 2)

Alternatively, a protected chenodeoxycholic derivative was subjected to treatment with KHMDS and Davies oxaziridine (Fiander and Schneider; Bioorganic and Medicinal Chemistry Letters, 1996, 637-642) to introduce the 23 functionality and give compound **9,** which was transformed by standard oxidation methods into the alpha keto ester **10.** Intramolecular directed CH-oxidation of **10** by *in situ* formation of a dioxirane ring using Oxone (Tang Y. C et al.; JACS, 1998, 6611-6612) (intermediate **11)** and subsequent elimination yielded the $\Delta^{16,17}$ olefin **12,** which was subjected to allylic oxidation to give compound **13.** Reduction of both the double bond and the ketone group in **13** was effected in one pot by catalytic hydrogenation. Finally hydrolysis of the protective groups in **14** and conjugation with taurine also gave the desired tauroconjugate tetrahydroxy bile acid **8.**

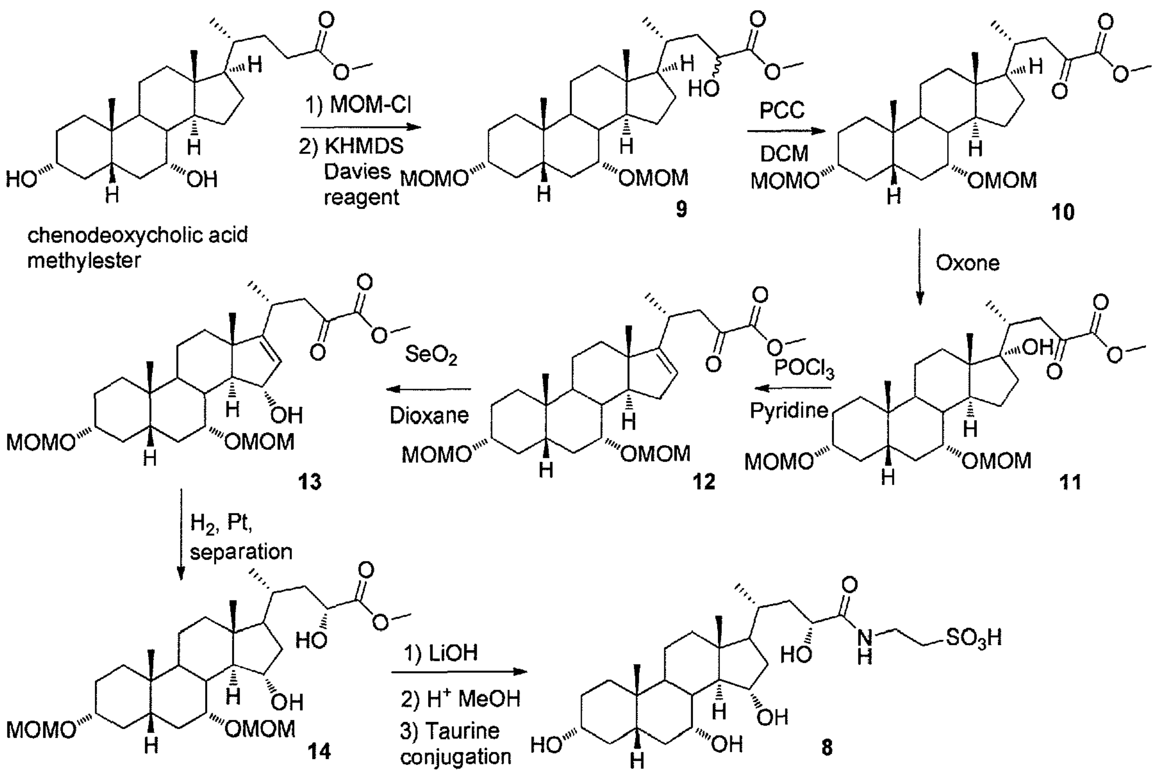


chenodeoxycholic acid methylester

9

10

11

12

13

14

8

**Scheme 2**

Example 3: Differentiation assay.

3T3L1 cells were grown to 60-70% confluence in a 96well dish and either left untreated or treated with the 3α,7β,15α,(23R)-tetrahydroxy-5β-24-oxocholanyl-amino ethansulfonic acid (see Example 1) at the indicated concentrations (10 or 100 μM). Cells were grown for an additional 48h after treatment before induction of differentiation using, dexamethasone, insulin and IBMX. Differentiation was quantified using high throughput imaging. The results are shown in Figure 1. The Y-axis is differentiation (in %). The X axis is the amount of 3α,7β,15α,(23R)-tetrahydroxy-5β-24-oxocholanyl-amino ethansulfonic acid (indicated as "tetraol") in μM.

## Claims

1. A compound of formula I for use as modulator of the ROR gamma receptor, having the general formula I or pharmaceutically acceptable salts or stereoisomers thereof

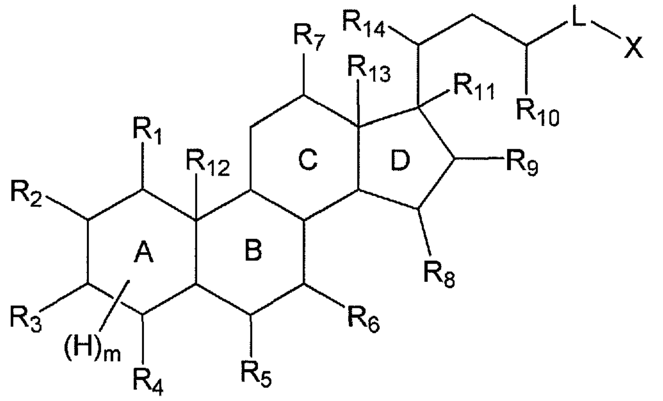

I

wherein

$R_1$, $R_2$, are independently of each other H, hal, -$OR_a$, -$SR_a$, -$NR_aR_b$, -$COOR_a$, -$CONR_aR_b$, wherein $R_a$ and $R_b$ are independently of each other H or (C1-6)alkyl, or $R_1$, $R_2$ form together with the C-atoms to which they are linked an epoxy group;
$R_3$, $R_4$, $R_5$, $R_6$, $R_7$, $R_8$, $R_9$, $R_{10}$, $R_{11}$ are independently of each other H, hal, -$OR_a$, -$SR_a$, -$NR_aR_b$, - $COOR_a$, -$CONR_aR_b$, oxo, thio wherein $R_a$ and $R_b$ are independently of each other H or (C1-6)alkyl, with the proviso that at least one of $R_8$, $R_9$, $R_{10}$, $R_{11}$ is -$OR_a$;
$R_{12}$, $R_{13}$, $R_{14}$ are independently of each other H, (C1-10)alkyl, wherein one or more non neighbouring $CH_2$ groups may be replaced with -O-, -S-, -CO-, -CO-O-, -O-CO-, -$NR_a$-, -CO-$NR_a$-, -$NR_a$-CO-, -C=C-, or -C≡C-; wherein $R_a$ is H or (C1-6)alkyl;
L is a linking group, such as a covalent bond or a straight-chain or branched C(1-12)alkyl, which is unsubstituted or substituted by at least one CN, hal, OH, $NR_aR_b$, $COOR_a$, $NO_2$, and wherein one or more of the non-adjacent $CH_2$ groups may independently be replaced by a group selected from -O-, -CO-, -CO-O-, -O-CO-, -$NR_a$-, -$NR_a$-CO-, -CO-$NR_a$-, - CH=CH-, -C≡C-, wherein $R_a$ and $R_b$ are independently of each other H or C(1-6)alkyl,
X is H, -$OR_a$, -$SR_a$, -$NR_aR_b$, -$COOR_c$, -$CONR_aR_c$; wherein $R_a$ and $R_b$ are independently of each other H or (C1-6)alkyl and $R_c$ is -H, -(C1-6)alkyl, -NH-$(CH_2)_n$-$CO_2R_a$ or -NH-$(CH_2)$p-$SO_3R_a$, wherein n, p is 1, 2, or 3 and $R_a$ is -H or -(C1-6)alkyl;
m is 0 to 5.

2. A compound according to claim 1 wherein $R_{12}$, $R_{13}$, $R_{14}$ are independently of each other H, (C1 -10)alkyl, more preferably methyl, ethyl, propyl, butyl, most preferably methyl.

3. A compound according to claims 1 or 2 wherein $R_1$, $R_2$ are independently of each other H, hal, -$OR_a$, -$COOR_a$, wherein $R_a$ is H or (C1-6)alkyl, or form together with the C-atoms to which they are linked an epoxy group.

4. A compound according to any preceding claim wherein $R_3$, $R_4$, $R_5$, $R_6$, $R_7$, $R_8$, $R_9$, and $R_{10}$ are independently of each other H, -$OR_a$, -$NR_aR_b$, -$COOR_a$, -$CONR_aR_b$ or oxo, wherein $R_a$ and $R_b$ are independently of each other H or (C1-6)alkyl, with the proviso that at least one of $R_8$, $R_9$, $R_{10}$ is -$OR_a$.

5. A compound according to any preceding claim, wherein at least three groups, preferably three or four groups, selected from $R_3$, $R_4$, $R_5$, $R_6$, $R_7$, $R_8$, $R_9$, , $R_{10}$ and $R_{11}$ are -$OR_a$ or oxo, with the proviso that at least one of $R_8$, $R_9$, $R_{10}$, $R_{11}$ is -$OR_a$.

6. A compound according to any preceding claim, wherein $R_4$ is H or hal.

7. A compound according to any preceding claim having formula II and pharmaceutically acceptable salts or stereoisomers thereof, preferably having formula IIa,

**8.**

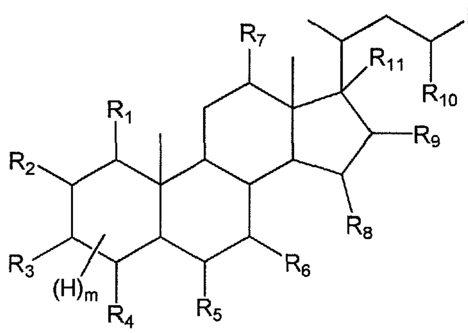

II

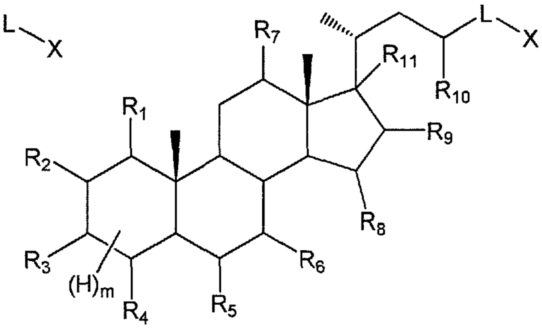

IIa

wherein

$R_1$, $R_2$, are independently of each other H, hal, $-OR_a$, $-SR_a$, $-NR_aR_b$, $-COOR_a$, $-CONR_aR_b$, wherein $R_a$ and $R_b$ are independently of each other H or (C1-6)alkyl, or $R_1$, $R_2$ form together with the C-atoms to which they are linked an epoxy group;

$R_3$, $R_5$, $R_6$, $R_7$, $R_8$, $R_9$, $R_{10}$, $R_{11}$ are independently of each other H, hal, $-OR_a$, $-SR_a$, $-NR_aR_b$, $-COOR_a$, $-CONR_aR_b$, oxo, thio wherein $R_a$ and $R_b$ are independently of each other H or (C1-6)alkyl, with the proviso that at least one of $R_8$, $R_9$, $R_{10}$, $R_{11}$ is $-OR_a$;

$R_4$ is H, hal,

L is a linking group, such as a covalent bond or straight-chain or branched C(1-12)alkyl, which is unsubstituted or substituted by at least one CN, hal, OH, $NR_aR_b$, $COOR_a$, $NO_2$, and wherein one or more of the non-adjacent $CH_2$ groups may independently be replaced by a group selected from -O-, -CO-, -CO-O-, -O-CO-, $-NR_a-$, $-NR_a-CO-$, $-CO-NR_a-$, - CH=CH-, -C≡C-, wherein $R_a$ and $R_b$ are independently of each other H or C(1-6)alkyl,

X is H, $-OR_a$, $-SR_a$, $-NR_aR_b$, $-COOR_c$, $-CONR_aR_c$; wherein $R_a$ and $R_b$ are independently of each other H or (C1-6)alkyl and $R_c$ is -H, -(C1-6)alkyl, $-NH-(CH_2)_n-CO_2R_a$ or $-NH-(CH_2)_p-SO_3R_a$, wherein n, p is 1, 2, or 3 and $R_a$ is -H or -(C1-6)alkyl;

m is 0 to 5.

**9.** A compound according to any preceding claim wherein L is a covalent bond or a straight-chain or branched C(1-12)alkyl, which is unsubstituted and wherein one or more of the non-adjacent $CH_2$ groups may independently be replaced by a group selected from -O-, - CO-, - CO-O-, preferably straight-chain or branched C(1-12)alkyl.

**10.** A compound according to any preceding claim having formula IIIa, IIIb, IIIc, IIId and all pharmaceutically acceptable salts or stereoisomers thereof,

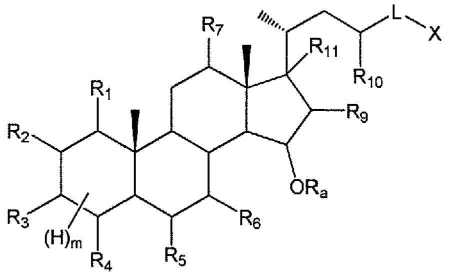

IIIa

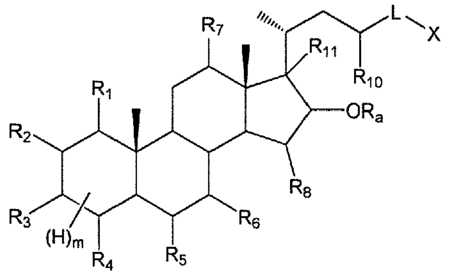

IIIb

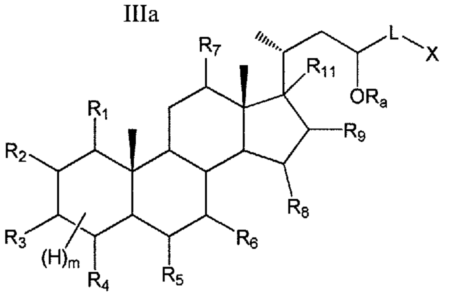

IIIc

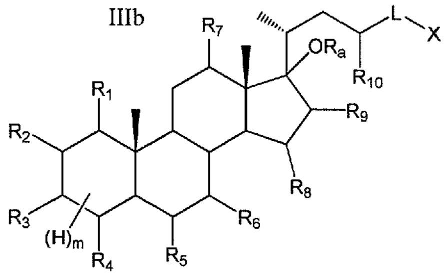

IIId

wherein

$R_1$, $R_2$, are independently of each other H, hal, $-OR_a$, $-SR_a$, $-NR_aR_b$, $-COOR_a$, $-CONR_aR_b$, wherein $R_a$ and $R_b$ are independently of each other H or (C1-6)alkyl, or $R_1$, $R_2$ form together with the C-atoms to which they are linked to an epoxy group;
$R_3$, $R_5$, $R_6$, $R_7$, $R_8$, $R_9$, $R_{10}$, $R_{11}$, are independently of each other H, hal, $-OR_a$, $-SR_a$, $-NR_aR_b$, - $COOR_a$, $-CONR_aR_b$, oxo, thio wherein $R_a$ and $R_b$ are independently of each other H or (C1-6)alkyl, with the proviso that at least one group, preferably two or three groups selected from of $R_1$, $R_2$, $R_3$, $R_4$, $R_5$, $R_6$, $R_7$, $R_8$, $R_9$, $R_{10}$, and $R_{11}$, are $-OR_a$;
$R_4$ is H, hal,
L is a covalent bond or straight-chain or branched C(1-12)alkyl, which is unsubstituted and wherein one or more of the non-adjacent $CH_2$ groups may independently be replaced by a group selected from -O-, -CO-, - CO-O;
X is H, $-OR_a$, $-SR_a$, $-NR_aR_b$, $-COOR_c$, $-CONR_aR_c$; wherein $R_a$ and $R_b$ are independently of each other H or (C1-6)alkyl and $R_c$ is -H, -(C1-6)alkyl, $-NH-(CH_2)_n-CO_2R_a$ or $-NH-(CH_2)p-SO_3R_a$, wherein n, p is 1, 2, or 3 and $R_a$ is -H or -(C1-6)alkyl;
$R_a$ is H or C(1-6)alkyl,
m is 0 to 5.

**11.** A compound according to claim 9, wherein (i) in compounds of formula IIIa two or three groups selected from of $R_1$, $R_3$, $R_5$, $R_6$, $R_7$, $R_9$, $R_{10}$ and $R_{11}$ are $-OR_a$; (ii) in compounds of formula IIIb two or three groups selected from of $R_1$, $R_3$, $R_5$, $R_6$, $R_7$, $R_8$, $R_{10}$ and $R_{11}$ are - $OR_a$; (iii) in compounds of formula IIIc two or three groups selected from of $R_1$, $R_3$, $R_5$, $R_6$, $R_7$, $R_8$, $R_9$, and $R_{11}$ are $-OR_a$, (iv) in compounds of formula IIId two or three groups selected from of $R_1$, $R_3$, $R_5$, $R_6$, $R_7$, $R_8$, $R_9$, and $R_{10}$ are $-OR_a$, wherein $R_a$ is H or (Cl-6)alkyl.

**12.** A compound according to claim 9 or 10, wherein (i) $R_3$ and $R_6$ are $-OR_a$, or (ii) $R_5$ and $R_6$ are $-OR_a$, or (iii) $R_3$ and $R_7$ are $-OR_a$, or (iv) $R_1$ and $R_3$ are $-OR_a$, or (v) $R_1$ and $R_5$ are - $OR_a$, or (vi) $R_1$ and $R_6$ are $-OR_a$, or (vii) $R_1$ and $R_7$ are $-OR_a$, or (viii) $R_6$ and $R_7$ are $-OR_a$, or (ix) $R_3$ and $R_5$ are $-OR_a$, or (x) $R_5$ and $R_7$ are $-OR_a$, wherein $R_a$ is H or (C1-6)alkyl.

**13.** A compound according to claim 9 or 10, wherein (i) $R_1$, $R_3$ and $R_6$ are $-OR_a$, or (ii) $R_3$, $R_6$ and $R_7$ are $-OR_a$, or (iii) $R_1$, $R_5$ and $R_6$ are $-OR_a$, or (iv) $R_5$, $R_6$ and $R_7$ are $-OR_a$, or (v) $R_1$, $R_3$ and $R_5$ are $-OR_a$, or (vi) $R_3$, $R_5$ and $R_6$ are $-OR_a$, or (vii) $R_1$, $R_3$ and $R_7$ are $-OR_a$, or (viii) $R_1$, $R_5$ and $R_7$ are $-OR_a$, or (ix) $R_1$, $R_6$ and $R_7$ are $-OR_a$, wherein $R_a$ is H or (C1-6)alkyl.

**14.** A compound according to any preceding claim, wherein $R_2$ and $R_4$ are H.

**15.** A compound according to any preceding claim having formulae Va, Vb, Vc, Vd or Ve, and all pharmaceutically acceptable salts or stereoisomers thereof,

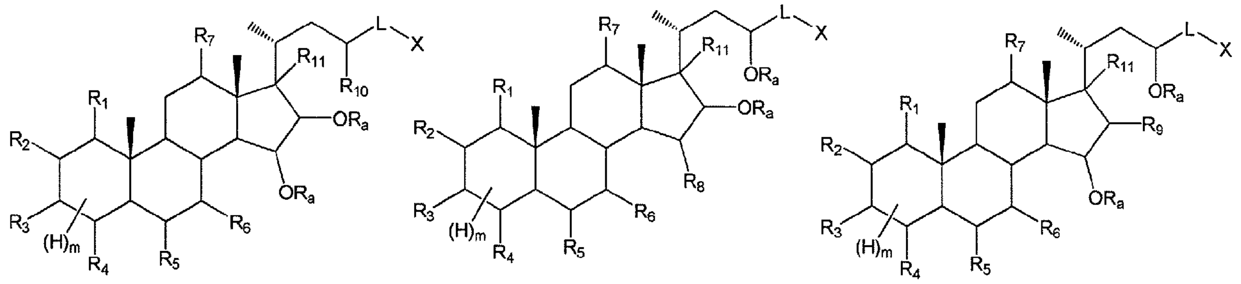

Va Vb Vc

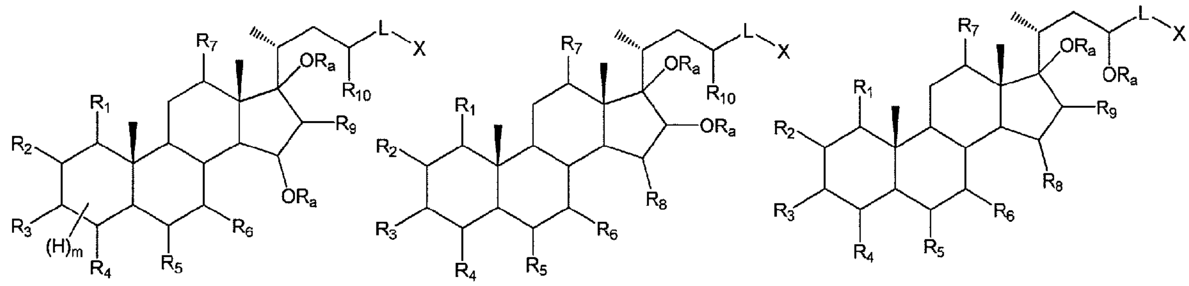

Vd Ve Vf

wherein

$R_1$, $R_2$, are independently of each other H, hal, $-OR_a$, $-SR_a$, $-NR_aR_b$, $-COOR_a$, $-CONR_aR_b$, wherein $R_a$ and $R_b$ are independently of each other H or (C1-6)alkyl, or $R_1$, $R_2$ form together with the C-atoms to which they are linked to an epoxy group;

$R_3$, $R_5$, $R_6$, $R_7$, $R_8$, $R_9$, $R_{10}$, $R_{11}$, are independently of each other H, hal, $-OR_a$, $-SR_a$, $-NR_aR_b$, - $COOR_a$, $-CONR_aR_b$, oxo, thio wherein $R_a$ and $R_b$ are independently of each other H or (C1-6)alkyl, wherein preferably one or two groups selected from of $R_1$, $R_2$, $R_3$, $R_4$, $R_5$, $R_6$, $R_7$, $R_8$, $R_9$, $R_{10}$, and $R_{11}$ are $-OR_a$;

$R_4$ is H, hal,

L is a covalent bond or a straight-chain or branched C(1-12)alkyl, which is unsubstituted and wherein one or more of the non-adjacent $CH_2$ groups may independently be replaced by a group selected from -O-, -CO-, - CO-O;

X is H, $-OR_a$, $-SR_a$, $-NR_aR_b$, $-COOR_c$, $-CONR_aR_c$; wherein $R_a$ and $R_b$ are independently of each other H or (C1-6)alkyl and $R_c$ is -H, -(C1-6)alkyl, $-NH-(CH_2)_n-CO_2R_a$ or $-NH-(CH_2)p-SO_3R_a$, wherein n, p is 1, 2, or 3 and $R_a$ is -H or -(C1-6)alkyl;

$R_a$ is H or C(1-6)alkyl,

m is 0 to 5.

**16.** A compound according to claim 14, wherein (i) in compounds of formula Va one or two groups selected from of $R_1$, $R_3$, $R_5$, $R_6$, $R_7$, $R_{10}$, and $R_{11}$ are $-OR_a$; (ii) in compounds of formula Vb one or two groups selected from of $R_1$, $R_3$, $R_5$, $R_6$, $R_7$, $R_8$ and $R_{11}$ are $-OR_a$; (iii) in compounds of formula Vc one or two groups selected from of $R_1$, $R_3$, $R_5$, $R_6$, $R_7$, $R_9$ and $R_{11}$ are $-OR_a$, (iv) in compounds of formula Vd one or two groups selected from of $R_1$, $R_3$, $R_5$, $R_6$, $R_7$, $R_9$ and $R_{10}$ are $-OR_a$, (v) in compounds of formula Ve one or two groups selected from of $R_1$, $R_3$, $R_5$, $R_6$, $R_7$, $R_8$ and $R_{10}$ are $-OR_a$, (vi) in compounds of formula Vf one or two groups selected from of $R_1$, $R_3$, $R_5$, $R_6$, $R_7$, $R_8$ and $R_9$ are $-OR_a$, wherein $R_a$ is H or (C1-6)alkyl..

**17.** A compound according to claim 14 or 15, wherein (i) $R_3$ and $R_6$ are $-OR_a$ or (ii) $R_5$ and $R_6$ are $-OR_a$, or (iii) $R_3$ and $R_7$ are $-OR_a$, or (iv) $R_1$ and $R_3$ are $-OR_a$, or (v) $R_1$ and $R_5$ are - $OR_a$, or (vi) $R_1$ and $R_6$ are $-OR_a$ or (vii) $R_1$ and $R_7$

are $-OR_a$, or (viii) $R_6$ and $R_7$ are $-OR_a$, or (ix) $R_3$ and $R_5$ are $-OR_a$, or (x) $R_5$ and $R_7$ are $-OR_a$, wherein $R_a$ is H or (C1-6)alkyl.

**18.** A compound according to any preceding claim having formula VIIa, VIIb, VIIc and all pharmaceutically acceptable salts or stereoisomers thereof,

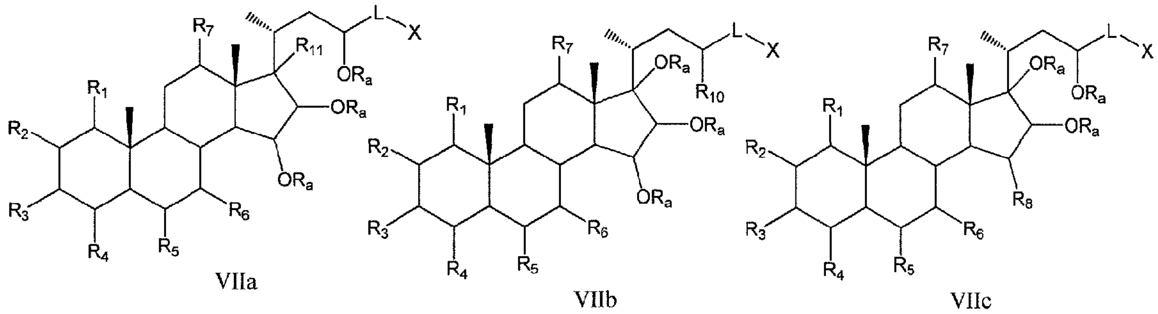

VIIa VIIb VIIc

wherein

$R_1$, $R_3$, $R_5$, $R_6$, $R_7$, $R_8$, $R_9$, $R_{10}$, $R_{11}$, are independently of each other H, hal, $-OR_a$, $-SR_a$, $-NR_aR_b$, $-COOR_a$, $-CONR_aR_b$, oxo, thio wherein $R_a$ and $R_b$ are independently of each other H or (C1-6)alkyl;
L is a covalent bond or a straight-chain or branched C(1-12)alkyl;
X is H, $-OR_a$, $-COOR_c$, $-CONR_aR_c$, wherein $R_a$ is H or (C1-6)alkyl and $R_c$ is -H, -(C1-6)alkyl, $-NH-(CH_2)_n-CO_2R_a$ or $-NH-(CH_2)_p-SO_3R_a$, wherein n, p is 1, 2, or 3 and $R_a$ is -H or -(C1-6)alkyl, and
$R_a$ is H or C(1-6)alkyl,
m is 0 to 5.

**19.** A compound according to claim 17, wherein one group selected from of $R_1$, $R_3$, $R_5$, $R_6$, $R_7$, $R_8$, $R_9$, $R_{10}$, $R_{11}$.

**20.** Compound according to any one of claims 1 to 18 for use in the treatment or prevention, suppression or amelioration of a disease mediated by the ROR gamma receptor in a subject in need thereof, preferably selected from the group consisting of diabetes and diabetes-related disorders, in particular type II diabetes.

**21.** Pharmaceutical composition comprising at least one compound according to any one of claims 1 to 18 and at least one pharmaceutically acceptable carrier, optionally comprising at least one further therapeutically active agent.

**22.** Use of at least one compound according to any one of claims 1 to 18 or a pharmaceutically acceptable salt thereof or a pharmaceutical composition according to claim 20, for the treatment or prevention of disorders, diseases or conditions responsive to the modulation of the ROR gamma receptor, preferably for the treatment or prevention of diabetes and diabetes-related disorders, preferably type II diabetes in a subject in need thereof.

**23.** Method for the treatment or prevention of disorders, diseases or conditions responsive to the modulation of the ROR gamma receptor in a subject in need thereof, which comprises administering to the subject a therapeutically or prophylactically effective amount of at least one compound according to any one of claims 1 to 18 or a pharmaceutically acceptable salt or stereoisomer thereof, optionally in combination with at least one further therapeutically active agent, or a pharmaceutical composition according to claim 20.

**24.** Method for the treatment or prevention of diabetes and diabetes-related disorders, preferably type II diabetes, in a subject in need thereof which comprises administering to said subject a therapeutically or prophylactically effective amount of at least one compound according to any one of claims 1 to 18 or a pharmaceutically acceptable salt thereof, optionally in combination with at least one further therapeutically active agent, or a pharmaceutical composition according to claim 20.

**25.** Kit comprising at least one compound according to any one of claims 1 to 18 or a pharmaceutically acceptable salt or stereoisomer thereof, or a pharmaceutical composition according to claim 20.

Figure 1

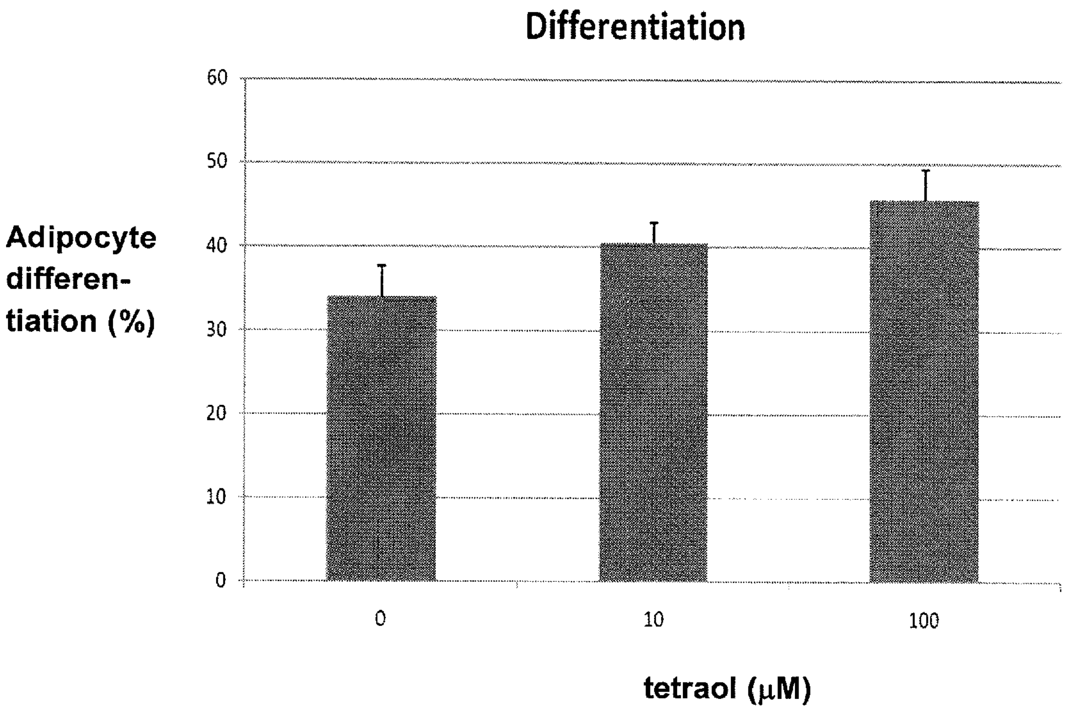
Differentiation
Adipocyte differen-tiation (%)
60
50
40
30
20
10
0
0
10
100
tetraol (μM)

Europäisches Patentamt
European Patent Office
Office européen des brevets

# EUROPEAN SEARCH REPORT

Application Number
EP 13 15 9736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MATOVIC, NICHOLAS J. ET AL: "The truth about false unicorn (Chamaelirium luteum): Total synthesis of 23R,24S-chiograsterol B defines the structure and stereochemistry of the major saponins from this medicinal herb", CHEMISTRY - A EUROPEAN JOURNAL , 17(27), 7578-7591, S7578/1-S7578/20 CODEN: CEUJED; ISSN: 0947-6539 DOI - DOI:10.1002/CHEM.201100503, 2011, XP8163233, * page 7579, column 1; figure 1 * * page 7579, column 1, paragraph 1 * ----- | 1-4, 6-12,14, 15, 20-23,25 | INV. A61K31/575 A61P3/10 |
| X | YASUKAWA KEN ET AL: "Relative inhibitory activity of bile acids against 12-O-tetradecanoylphorbol-13-acetate-induced inflammation, and chenodeoxycholic acid inhibition of tumour promotion in mouse skin two-stage carcinogenesis", JOURNAL OF PHARMACY AND PHARMACOLOGY, JOHN WILEY & SONS LTD, LONDON; GB, vol. 61, no. 8, 1 August 2009 (2009-08-01) , pages 1051-1056, XP008119235, ISSN: 0022-3573, DOI: 10.1211/JPP/61.08.0009 * figure 1; compounds 15-23,38-41 * * table 1; compounds 15-23,38-41 * * page 1051, paragraph 1 - paragraph 2 * ----- | 1-4, 6-10,14, 20-23,25 | |
| | -/-- | | TECHNICAL FIELDS SEARCHED (IPC) |
| | | | A61K |

The present search report has been drawn up for all claims

3

| Place of search | Date of completion of the search | Examiner |
|---|---|---|
| The Hague | 2 July 2013 | Bonzano, Camilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 9736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JUN R. HUH ET AL: "Digoxin and its derivatives suppress TH17 cell differentiation by antagonizing ROR[gamma]t activity", NATURE, vol. 472, no. 7344, 27 March 2011 (2011-03-27), pages 486-490, XP55042051, ISSN: 0028-0836, DOI: 10.1038/nature09978 * abstract * * figure 3 * ----- | 1-4, 6-10,14, 20-23,25 | |
| A | CHALLINOR VICTORIA L ET AL: "Structure and Bioactivity of Steroidal Saponins Isolated from the Roots of Chamaelirium luteum (False Unicorn)", JOURNAL OF NATURAL PRODUCTS, AMERICAN CHEMICAL SOCIETY, US , vol. 75, no. 8 24 August 2012 (2012-08-24), pages 1469-1479, XP008163234, ISSN: 0163-3864, DOI: 10.1021/NP300393Y Retrieved from the Internet: URL:http://pubs.acs.org/journals/jnprdf/index.html [retrieved on 2012-08-10] ----- | 1 | |
| | -/-- | | TECHNICAL FIELDS SEARCHED (IPC) |

The present search report has been drawn up for all claims

3

| Place of search | Date of completion of the search | Examiner |
|---|---|---|
| The Hague | 2 July 2013 | Bonzano, Camilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 9736

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | NARESH KUMAR ET AL: "The Benezenesulfonamide T0901317 [N-(2,2,2-Trifluoroethyl)-N-[4-[2,2,2-trifluoro-1-hydroxy-1-(trifluoromethyl)ethyl]phenyl]-benzenesulfonamide] is a Novel Retinoic Acid Receptor-Related Orphan Receptor-alpha/gamma Inverse Agonist", MOLECULAR PHARMACOLOGY, AMERICAN SOCIETY FOR PHARMACOLOGY AND EXPERIMENTAL THERAPEUTICS, US, vol. 77, no. 2, 1 February 2010 (2010-02-01), pages 228-236, XP002645391, ISSN: 0026-895X, DOI: 10.1124/MOL.109.060905 [retrieved on 2009-11-03] * abstract * ----- | 1 | |
| | | | TECHNICAL FIELDS SEARCHED (IPC) |

The present search report has been drawn up for all claims

3

| Place of search | Date of completion of the search | Examiner |
|---|---|---|
| The Hague | 2 July 2013 | Bonzano, Camilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6414002 B
- WO 9736579 A
- WO 9728149 A
- WO 0121577 A
- WO 0121169 A
- WO 0196302 A
- WO 0168609 A
- WO 0251232 A
- WO 0251838 A


### Non-patent literature cited in the description


- **BECKER-ANDREE.** *Biochem. Biophys. Res. Commun.,* 1993, vol. 194, 1371
- **ANDRE et al.** *Gene,* 1998, vol. 516, 277
- **HE et al.** *Immunity,* 1998, vol. 9, 797
- **MANGELSDORF et al.** *Cell,* 1995, vol. 83, 835
- **KIEC-KONONOWICZ, K. et al.** *Pharmazie,* 2000, vol. 55, 349-55
- **LAZEWSKA, D. et al.** *Pharmazie,* 2001, vol. 56, 927-32
- **SASSE, A. et al.** *Arch. Pharm.,* 2001, vol. 334, 45-52
- **REIDEMEISTER, S. et al.** *Pharmazie,* 2000, vol. 55, 83-6
- **SASSE, A. et al.** *J. Med. Chem.,* 2000, vol. 43, 3335-43
- **OLITVAK, D.A. et al.** *Dig. Dis. Sci.,* 1999, vol. 44 (3), 643-48
- **BATTERHAM et al.** *J. Clin. Endocrinol. Metab.,* 2003, vol. 88, 3989-3992
- **GREENE, T ; WUTS, P. G. M.** Protective Groups in Organic Synthesis. John Wiley and Sons, Inc, 1991
- **MITRA U.** *Steroids,* 2010, 506-512
- **OSAWA R.** *Bulletin of the Chemical Society of Japan,* 1962, 158-162
- **SCHROEPFER ; PARISH.** *Chemistry and Physics of Lipids,* 1980, 282-288
- **TOHMA M. et al.** *Chem. Pharm. Bull.,* 1989, 2147-2152
- **FIANDER ; SCHNEIDER.** *Bioorganic and Medicinal Chemistry Letters,* 1996, 637-642
- **PELLICCIARI R.** *J. Med Chem.,* 1985, 239-242
- **TANG Y. C et al.** *JACS,* 1998, 6611-6612